(19) Europäisches Patentamt
European Patent Office
Office européen des brevets

(11) Veröffentlichungsnummer: **0 109 636**
**B1**

# (12) EUROPÄISCHE PATENTSCHRIFT

(45) Veröffentlichungstag der Patentschrift: **18.01.89**

(51) Int. Cl.⁴: **C 07 D 223/16**, C 07 D 243/04, A 61 K 31/55 // C07C103/50

(21) Anmeldenummer: **83111348.5**

(22) Anmeldetag: **14.11.83**

(54) **Benzazepinderivate, ihre Herstellung und ihre Verwendung als Arzneimittel.**

(30) Priorität: **18.11.82 DE 3242599**

(43) Veröffentlichungstag der Anmeldung: **30.05.84 Patentblatt 84/22**

(45) Bekanntmachung des Hinweises auf die Patenterteilung: **18.01.89 Patentblatt 89/3**

(84) Benannte Vertragsstaaten: **AT BE CH DE FR IT LI LU NL SE**

(56) Entgegenhaltungen:
**EP-A- 0 065 229**
**DE-A- 2 639 718**
**US-A- 3 474 090**
**US-A- 3 780 023**
**US-A- 4 210 749**

(73) Patentinhaber: **Dr. Karl Thomae GmbH, Postfach 1755, D-7950 Biberach (Riss) (DE)**

(72) Erfinder: **Reiffen, Manfred, Dr. Dipl.-Chem., Amriswilstrasse 7, D-7950 Biberach 1 (DE)**
Erfinder: **Heider, Joachim, Dr. Dipl.-Chem., Am Hang 3, D-7951 Warthausen 1 (DE)**
Erfinder: **Austel, Volkhard, Austel, Dr. Dipl.-Chem., Kapellenweg 7, D-7950 Biberach 1 (DE)**
Erfinder: **Hauel, Norbert, Dr. Dipl.-Chem., Händelstrasse 12, D-7950 Biberach 1 (DE)**
Erfinder: **Kobinger, Walter, Prof. Dr., Beighofergasse 27, A-1121 Wien (AT)**
Erfinder: **Lillie, Christian, Dr., Hansl-Niese-Weg 12, A-1121 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

In der britischen Patentschrift 1 548 844 wird u.a. die Verbindung der Formel

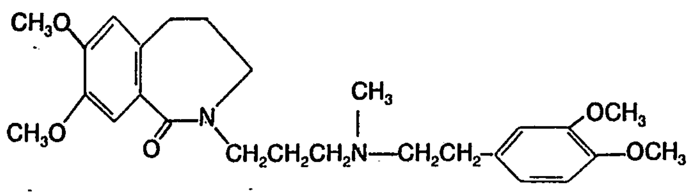

und deren physiologisch verträgliche Säureadditionssalze beschrieben, welche wertvolle pharmakologische Eigenschaften aufweist, nämlich neben einer milden blutdrucksenkenden Wirkung insbesondere eine selektive herzfrequenzsenkende Wirkung.

Ausserdem werden in der nicht vorveröffentlichten EP-A-0 065 229 bereits Benzazepinderivate, welche wertvolle pharmakologische Eigenschaften aufweisen, nämlich insbesondere eine herzfrequenzsenkende Wirkung, beschrieben.

Überraschenderweise wurde nun gefunden, dass die neuen Benzazepinderivate der allgemeinen Formel I

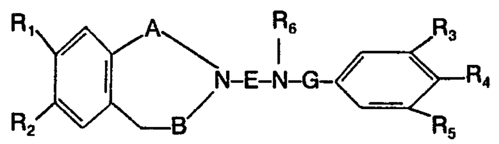 (I)

sowie

1-(7,8-Dimethoxy-1,3,4,5-tetrahydro-2H-3-benzazepin-2-on-3-yl)-3-[N-methyl-N-(2-phenylethyl)-amino]-propan und

1-(7,8-Dimethoxy-1,3,4,5-tetrahydro-2H-3-benzazepin-2-on-3-yl)-3-[N-methyl-N-(2-(4-amino-3-nitro-phenyl)-ethyl]-amino]-propan

wie die Benzazepinderivate der EP-A-0 065 229 überlegene, pharmakologische Eigenschaften aufweisen, nämlich bei einer längeren Wirkungsdauer und geringeren Nebenwirkungen, insbesondere eine stärkere herzfrequenzsenkende Wirkung.

Gegenstand der Erfindung sind somit die obigen neuen Benzazepinderivate, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel I bedeutet

A eine $-CH_2-CH_2-$ oder $-CH=CH-$Gruppe und

B die –CS-Gruppe oder auch, wenn

G eine n-Alkylengruppe mit 3 bis 5 Kohlenstoffatomen oder eine Methylen-n-hydroxyalkylengruppe mit 1 bis 3 Kohlenstoffatomen im Alkylenteil darstellt, wobei die Methylengruppe mit dem Stickstoffatom verknüpft ist, die –CO-Gruppe oder

A eine

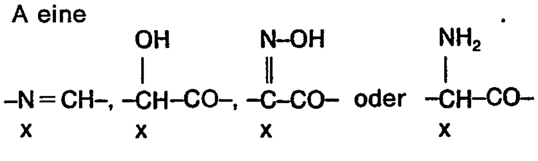

Gruppe

oder auch die –COCO-Gruppe, wenn

G eine n-Alkylengruppe mit 3 bis 5 Kohlenstoffatomen oder eine Methylen-n-hydroxyalkylengruppe mit 2 bis 3 Kohlenstoffatomen im Alkylenteil darstellt, wobei die Methylengruppe mit dem Stickstoffatom verknüpft ist, und

B die $-CH_2$-Gruppe, wobei das mit x gekennzeichnete Kohlenstoffatom jeweils mit dem Phenylkern verknüpft sein muss, E eine n-Propylengruppe,

G eine n-Alkylengruppe mit 2 bis 5 Kohlenstoffatomen oder eine Methylen-n-hydroxyalkylengruppe mit 1 bis 3 Kohlenstoffatomen im Alkylenteil, wobei die Methylengruppe mit dem Stickstoffatom verknüpft ist,

$R_1$ und $R_2$ jeweils eine Methoxygruppe,

$R_3$ ein Wasserstoff-, Fluor-, Chlor- oder Bromatom, eine Methoxy- oder Nitrogruppe,

$R_4$ ein Wasserstoffatom, eine Methoxy-, Amino-, Methylamino- oder Dimethylaminogruppe,

$R_5$ ein Wasserstoff-, Chlor- oder Bromatom und

$R_6$ ein Wasserstoffatom oder eine Methylgruppe.

Für die bei der Definition der Reste $R_3$ bis $R_6$ und G eingangs erwähnten Bedeutungen kommt

für $R_3$ die des Wasserstoff-, Fluor-, Chlor- oder Bromatoms, der Methoxy- oder Nitrogruppe,

für $R_4$ die des Wasserstoffatoms, der Methoxy-, Amino-, Methylamino- oder Dimethylaminogruppe,

für $R_5$ die des Wasserstoff-, Chlor- oder Bromatoms,

für $R_6$ die des Wasserstoffatoms oder der Methylgruppe und

für G die der

Ethylen-, 1-Methyl-ethylen-,
2-Ethyl-ethylen-,
1-Propyl-ethylen-, 2-Methyl-ethylen-,
n-Propylen-, n-Butylen-, n-Pentylen-,
1-Methyl-n-propylen-, 1-Methyl-butylen-,
1-Ethyl-n-propylen-, 2-Ethyl-n-propylen-,
1-Methyl-n-butylen-, 2-Hydroxy-ethyl-,
2-Hydroxy-n-propylen- oder 3-Hydroxy-n-propylen-

gruppe

in Betracht.

Bevorzugte Verbindungen sind jedoch die Verbindungen der allgemeinen Formel I, in der

E wie eingangs definiert ist,

A die $-CH_2CH_2$-Gruppe,

B die –CO- oder –CS-Gruppe,

$R_1$ und $R_2$ jeweils eine Methoxygruppe,

$R_3$ ein Wasserstoff-, Fluor-, Chlor- oder Bromatom oder eine Methoxygruppe,

$R_4$ eine Methoxy-, Amino-, Methylamino- oder Dimethylaminogruppe,

$R_5$ ein Wasserstoff-, Chlor- oder Bromatom,

$R_6$ die Methylgruppe und

G eine n-Alkylengruppe mit 3 bis 5 Kohlenstoffatomen oder

OH
|
die $-CH_2-CH$-Gruppe oder auch, wenn B die –CS-Gruppe darstellt, die $-CH_2CH_2$-Gruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel I sind diejenigen, in denen

E wie eingangs definiert ist,

A die $-CH_2CH_2$-Gruppe und

B die –CS-Gruppe oder auch, wenn G eine n-Alkylengruppe mit 3 bis 5 Kohlenstoffatomen darstellt, die –CO-Gruppe oder

OH
|
A die –CH–CO-Gruppe und

B die $-CH_2$-Gruppe,

$R_1$ und $R_2$ je eine Methoxygruppe,

$R_3$ eine Methoxygruppe, ein Wasserstoff- oder Chloratom,

$R_4$ eine Methoxy-, Amino- oder Dimethylaminogruppe,

$R_5$ ein Wasserstoff- oder Chloratom,

$R_6$ eine Methylgruppe,

G eine n-Alkylengruppe mit 3 bis 5 Kohlenstoffatomen oder

OH
|
eine –CH–CO-Gruppe oder auch, wenn B die –CS-Gruppe oder wenn

OH
|
A die –CH–CO-Gruppe darstellt, die $-CH_2CH_2$-Gruppe bedeuten, und deren physiologisch verträgliche Säureadditionssalze mit anorganischen oder organischen Säuren.

Erfindungsgemäss erhält man die neuen Verbindungen nach folgenden Verfahren:

a) Umsetzung einer Verbindung der allgemeinen Formel II

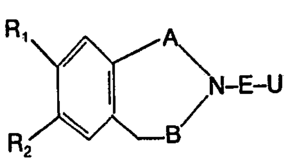

(II)

mit einer Verbindung der allgemeinen Formel III

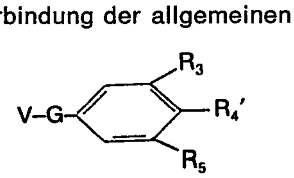

(III)

in denen

$R_1$, $R_2$, $R_3$, $R_5$, A, B, E und G wie eingangs definiert sind,

$R_4'$ eine durch einen Schutzrest geschützte Amino- oder Methylaminogruppe darstellt oder die für $R_4$ eingangs erwähnten Bedeutungen besitzt,

einer der Reste U oder V die $R_6$-NH-Gruppe darstellt, wobei $R_6$ wie eingangs definiert ist, und

der andere der Reste U oder V eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyl- oder Ethoxysulfonyloxygruppe, darstellt und gegebenenfalls anschliessende Abspaltung eines verwendeten Schutzrestes.

Die Umsetzung wird zweckmässigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuss der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmässigerweise bei Temperaturen zwischen 0 und 150 °C, vorzugsweise bei Temperaturen zwischen 50 und 120 °C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschliessende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z.B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Raumtemperatur zwischen 0 und 50 °C, vorzugsweise jedoch bei Raumtemperatur, und einem Wasserstoffdruck von 1 bis 7 bar, vorzugsweise jedoch von 3 bis 5 bar.

b) Zur Herstellung einer Verbindung der allgemeinen Formel I, in der A eine $-CH_2-CH_2-$, $-CH=CH-$, $-COCO-$ oder $-N=CH$-Gruppe und B eine $-CH_2-$ oder $-CO$-Gruppe darstellen:

Umsetzung einer Verbindung der allgemeinen Formel IV

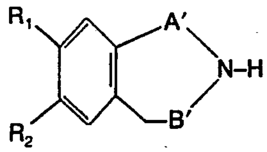

(IV)

in der

$R_1$ und $R_2$ wie eingangs definiert sind,

A′ eine $-CH_2-CH_2-$, $-CH=CH-$, $-COCO-$ oder $-N=CH$-Gruppe und

B′ eine $-CH_2-$ oder $-CO$-Gruppe bedeuten, mit einer Verbindung der allgemeinen Formel V

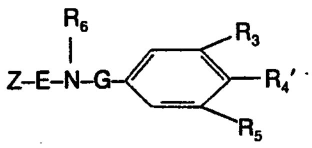

(V)

in der

$R_3$, $R_5$, $R_6$, E und G wie eingangs definiert sind,

$R_4'$ eine durch eine Schutzgruppe geschützte Amino- oder Methylaminogruppe darstellt oder die für $R_4$ eingangs erwähnten Bedeutungen besitzt und

Z eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, bedeutet und gegebenenfalls anschliessende Abspaltung eines verwendeten Schutzrestes.

Die Umsetzung wird gegebenenfalls in einem Lösungsmittel oder Lösungsmittelgemisch, z.B. in Aceton, Dimethylformamid, Aceton/Dimethylformamid, Dimethylsulfoxid oder Chlorbenzol, und zweckmässigerweise je nach der Reaktionsfähigkeit des Restes Z bei Temperaturen zwischen 0 und 150 °C, vorzugsweise jedoch bei der Siedetemperatur des verwendeten Lösungsmittels, durchgeführt. Vorteilhaft ist die Gegenwart eines säurebindenden Mittels, wie z.B. eines Alkoholats wie Natriummethylat, eines Alkalihydroxids wie Natriumhydroxid, eines Alkalicarbonats wie Kaliumcarbonat, eines Alkaliamids wie Natriumamid, eines Alkalihydrids wie Natriumhydrid oder einer tertiären organischen Base wie Triethylamin oder Pyridin, oder eines Reaktionsbeschleunigers wie beispielsweise Kaliumjodid.

Die gegebenenfalls anschliessende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes kann jedoch auch hydrogenolytisch erfolgen, z.B. mit Wasserstoff in Gegenwart eines Katalysators wie Palladium/Kohle, in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

c) Zur Herstellung einer Verbindung der allgemeinen Formel I,

$NH_2$
|
in der A keine $-CH-CO$-Gruppe darstellt:

Umsetzung einer Verbindung der allgemeinen Formel VI

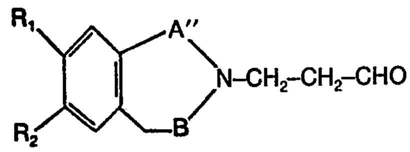

(VI)

in der

B, $R_1$ und $R_2$ wie eingangs definiert sind, und

A″ eine $-CH_2-CH_2-$, $-CH=CH-$, $-N=CH-$,

OH NOH
| ‖
$-CH-CO-$ oder $-C-CO$-Gruppe darstellt, mit einem Amin der allgemeinen Formel VII

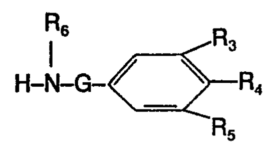

(VII)

in der

G und $R_3$ bis $R_6$ wie eingangs definiert sind, in Gegenwart eines Reduktionsmittels.

Die Umsetzung wird zweckmässigerweise in einem geeigneten Lösungsmittel oder Lösungsmittelgemisch wie Methanol, Ethanol, Ethanol/Essigsäureethylester oder Dioxan bei Temperaturen zwischen 0 und 100 °C, vorzugsweise jedoch bei Temperaturen zwischen 20 und 80 °C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6–7 und bei Raumtemperatur oder zur Herstellung von Verbindungen der allgemeinen Formel I, in der $R_6$ ein Wasserstoffatom darstellt, in Gegenwart eines Hydrierungskatalysators, z.B. mit Wasserstoff in Gegenwart von Palladium/Kohle bei einem Wasserstoffdruck von 5 bar, durchgeführt. Hierbei können gegebenenfalls vorhandene Doppelbindungen aufhydriert werden.

d) zur Herstellung einer Verbindung der allgemeinen Formel I, in der A keine

$NH_2$
|
$-CH-CO$-Gruppe darstellt:

Umsetzung einer Verbindung der allgemeinen Formel VIII

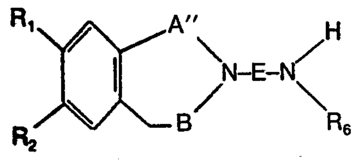
(VIII)

in der

B, E, $R_1$, $R_2$ und $R_6$ wie eingangs definiert sind und A″ eine $-CH_2-CH_2-$, $-CH=CH-$, $-N=CH-$,

OH NOH
| ‖
–CH–CO- oder –C–CO-Gruppe darstellt, mit einer Verbindung der allgemeinen Formel IX

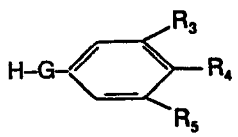
(IX)

in der

$R_3$ bis $R_5$ und G wie eingangs definiert sind, wobei jedoch im Rest G die endständige $-CH_2$-Gruppe durch eine Carbonylgruppe ersetzt ist, in Gegenwart eines Reduktionsmittels.

Die Umsetzung wird zweckmässigerweise in einem geeigneten Lösungsmittel oder Lösungsmittelgemisch wie Methanol, Ethanol, Ethanol/Essigsäureethylester oder Dioxan bei Temperaturen zwischen 0 und 100 °C, vorzugsweise jedoch bei Temperaturen zwischen 20 und 80 °C, durchgeführt.

Besonders vorteilhaft wird die reduktive Aminierung in Gegenwart eines komplexen Metallhydrids wie Lithium- oder Natriumcyanborhydrid vorzugsweise bei einem pH-Wert von 6–7 und bei Raumtemperatur oder zur Herstellung von Verbindungen der allgemeinen Formel I, in der $R_6$ ein Wasserstoffatom darstellt, in Gegenwart eines Hydrierungskaralysators, z. B. mit Wasserstoff in Gegenwart von Palladium/Kohle bei einem Wasserstoffdruck von 5 bar, durchgeführt. Hierbei können gegebenenfalls vorhandene Doppelbindungen aufhydriert werden.

e) Zur Herstellung einer Verbindung der allgemeinen Formel I, in der A die $-CH_2-CH_2$-Gruppe und B die Methylen- oder Carbonylgruppe darstellen:

Hydrierung einer Verbindung der allgemeinen Formel X

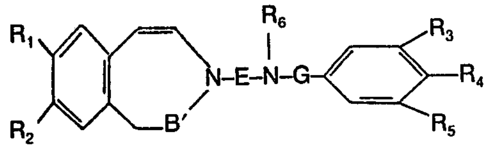
(X)

in der

$R_1$ bis $R_6$, E und G wie eingangs definiert sind und

B′ die Methylen- oder Carbonylgruppe darstellt.

Die Hydrierung wird in einem Lösungsmittel oder Lösungsmittelgemisch wie Methanol, Ethanol, Essigsäureethylester oder Eisessig mit katalytisch angeregtem Wasserstoff, z. B. mit Wasserstoff in Gegenwart von Platin oder Palladium/Kohle, bei einem Wasserstoffdruck von 1 bis 7 bar, vorzugsweise jedoch von 3 bis 5 bar, und bei Temperaturen zwischen 0 und 75 °C, vorzugsweise jedoch bei Temperaturen zwischen 20 und 50 °C, durchgeführt.

f) Zur Herstellung einer Verbindung der allgemeinen Formel I, in der B die –CS-Gruppe darstellt:

Umsetzung einer Verbindung der allgemeinen Formel XI

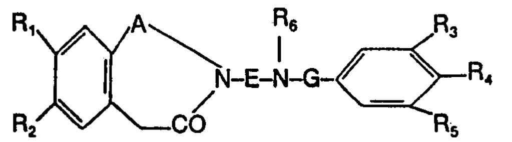
(XI)

in der

$R_1$ bis $R_6$, A, E und G wie eingangs definiert sind, mit einem schwefeleinführenden Mittel.

Die Umsetzung wird mit einem schwefeleinführenden Mittel wie Phosphorpentasulfid oder 2,4-Bis(4-methoxyphenyl)-1,2-dithia-2,4-diphosphetan-2,4-disulfid zweckmässigerweise in einem Lösungsmittel wie Toluol oder Xylol bei Temperaturen zwischen 50 und 150 °C, z. B. bei der Siedetemperatur des Reaktionsgemisches, durchgeführt.

g) Zur Herstellung einer Verbindung der allgemeinen Formel I, in der A eine

OH
|
–CH–CO-Gruppe darstellt:

Reduktion einer Verbindung der allgemeinen Formel XII

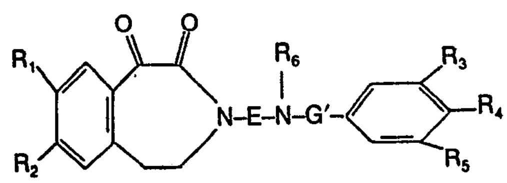
(XII)

in der

$R_1$ bis $R_6$ und E wie eingangs definiert sind und

G′ eine n-Alkylengruppe mit 2 bis 5 Kohlenstoffatomen, in der eine Methylengruppe durch eine Carbonylgruppe ersetzt sein kann, oder eine Methylen-n-hydroxyalkylengruppe mit 1 bis 3 Kohlenstoffatomen im Alkylenteil, wobei die Methylengruppe mit dem Stickstoffatom verknüpft ist, darstellt.

Die Umsetzung wird in Gegenwart eines geeigneten Reduktionsmittels wie einem Metallhydrid, z. B. Natriumborhydrid oder Lithiumaluminiumhydrid, in einem geeigneten Lösungsmittel wie Wasser/Methanol, Methanol/Ether, Tetrahydrofuran, Dioxan oder Ether/Tetrahydrofuran bei Temperaturen zwischen 0 und 80 °C, vorzugsweise jedoch bei Temperaturen zwischen 15 und 40 °C, durchgeführt.

h) Zur Herstellung einer Verbindung der allgemeinen Formel I, in der A eine

$NH_2$
|
–CH–CO– oder

NOH
|
–C–CO-Gruppe darstellt:

Umsetzung einer Verbindung der allgemeinen Formel XIII

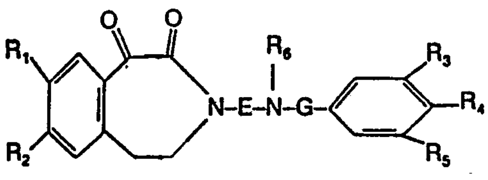

(XIII)

in der

$R_1$ bis $R_6$, E und G wie eingangs definiert sind, mit einer Verbindung der allgemeinen Formel XIV

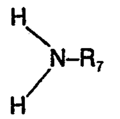

(XIV)

in der

$R_7$ ein Wasserstoffatom oder die Hydroxygruppe darstellt, und gegebenenfalls anschliessende Reduktion.

Die Umsetzung wird zweckmässigerweise in einem Lösungsmittel wie Ethanol, Dioxan oder Glykol oder in der Schmelze bei erhöhten Temperaturen, z.B. bei Temperaturen zwischen 50 und 175 °C, durchgeführt. Die gegebenenfalls anschliessende Reduktion wird mit einem Reduktionsmittel wie einem komplexen Metallhydrid, z.B. Lithiumaluminiumhydroxid, mit katalytisch angeregtem Wasserstoff, z.B. mit Wasserstoff in Gegenwart eines Hydrierungskatalysators wie Platin oder Palladium/Kohle bei einem Wasserstoffdruck von 1 bis 7 bar, vorzugsweise jedoch von 3 bis 5 bar, oder mit Hydrazin/Raney-Nickel in einem geeigneten Lösungsmittel wie Methanol, Ethanol, Essigsäureethylester oder Eisessig bei Temperaturen zwischen 0 und 100 °C, vorzugsweise jedoch bei Temperaturen zwischen 20 und 50 °C, durchgeführt.

i) Zur Herstellung einer Verbindung der allgemeinen Formel I, in der A die –N=CH-Gruppe und $R_6$ eine Methylgruppe darstellen:

Umsetzung einer Verbindung der allgemeinen Formel XV

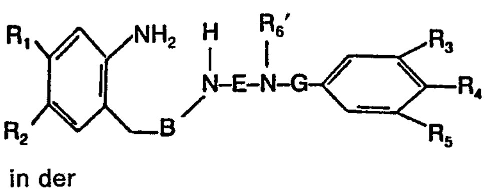

(XV)

in der

$R_1$ bis $R_5$, B, E und G wie eingangs definiert sind und $R_6'$ eine Methylgruppe darstellt, wobei gegebenenfalls vorhandene $NH_2$- oder NH-Gruppen geschützt sein können, mit einem Orthoameisensäureester und gegebenenfalls anschliessende Abspaltung eines verwendeten Schutzrestes.

Die Umsetzung wird vorzugsweise in einem Lösungsmittel wie Ethanol, Toluol, Dimethoxyethan oder in einem Überschuss des eingesetzten Orthoesters bei Temperaturen zwischen 100 und 200 °C durchgeführt.

Desweiteren kann es von Vorteil sein, wenn gegebenenfalls vorhandene $NH_2$- oder NH-Gruppen während der Umsetzung durch Schutzgruppen, z.B. durch Acetyl-, Benzoyl-, Ethoxycarbonyl- oder Benzylgruppen, geschützt werden.

Die gegebenenfalls während der Umsetzung verwendeten Schutzreste werden anschliessend wieder abgespalten, z.B. Acylreste vorzugsweise hydrolytisch in Gegenwart einer Säure oder Base bzw. Benzylreste vorzugsweise hydrogenolytisch, z.B. mit Wasserstoff in Gegenwart eines Hydrierungskatalysators wie Palladium/Kohle oder Platin.

k) Zur Herstellung einer Verbindung der allgemeinen Formel I, in der A keine –COCO-Gruppe und G eine Methylen-n-hydroxy-alkylengruppe darstellt:

Reduktion einer Verbindung der allgemeinen Formel XVI

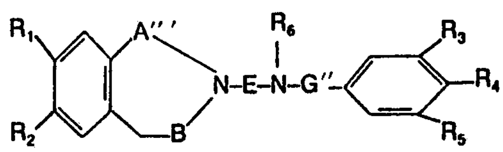

(XVI)

in der

$R_1$ bis $R_6$, B und E wie eingangs definiert sind,

A‴ mit Ausnahme der –COCO-Gruppe die für A eingangs erwähnten Bedeutungen besitzt und

G″ eine Methylen-n-alkylengruppe darstellt, wobei der Alkylenteil 1 bis 3 Kohlenstoffatome enthält und eine Methylengruppe des Alkylenteils durch eine Carbonylgruppe ersetzt ist.

Die Reduktion wird vorzugsweise mit einem Metallhydrid wie Natriumborhydrid in einem geeigneten Lösungsmittel wie Ethanol, Ethanol/Wasser, Methanol oder Isopropanol bei Temperaturen zwischen 0 und 50 °C, vorzugsweise jedoch bei Temperaturen zwischen 10 und 25 °C, durchgeführt.

1) Zur Herstellung einer Verbindung der allgemeinen Formel I, in der $R_3$ eine Nitrogruppe und $R_4$ eine Aminogruppe darstellen:

Hydrolyse einer Verbindung der allgemeinen Formel XVII

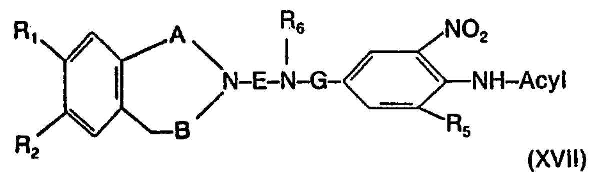

(XVII)

in der

$R_1$, $R_2$, $R_5$, $R_6$, A, B, E und G wie eingangs definiert sind und

Acyl einen Acylrest darstellt.

Die Abspaltung eines verwendeten Acylrestes erfolgt vorzugsweise hydrolytisch in einem wässrigen Lösungsmittel, z.B. in Wasser, Isopropanol/

Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Umsetzung wird jedoch vorzugsweise in Gegenwart einer alkoholischen Säure, z.B. mit methanolischer oder ethanolischer Salzsäure, durchgeführt.

m) Zur Herstellung einer Verbindung der allgemeinen Formel I, in der A eine $-CH_2-CH_2-$, $-CH=CH-$, $-N=CH-$ oder $-COCO-$Gruppe und einer der Reste $R_3$ oder $R_4$ eine Methoxy-, Methylamino- oder Dimethylaminogruppe oder $R_6$ eine Methylgruppe darstellen:

Umsetzung einer Verbindung der allgemeinen Formel XVIII

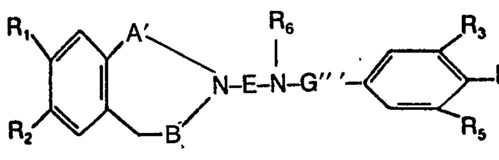

(XVIII)

in der

$R_1$ bis $R_6$, B und E wie eingangs definiert sind, wobei jedoch mindestens einer der Reste $R_1$ bis $R_4$ eine Hydroxygruppe oder $R_4$ eine Amino- oder Methylaminogruppe oder $R_6$ ein Wasserstoffatom darstellen muss,

A′ eine $-CH_2-CH_2-$, $-CH=CH-$, $-N=CH-$ oder $-COCO-$Gruppe und

G‴ eine n-Alkylengruppe mit 2 bis 5 Kohlenstoffatomen darstellt, mit einer Verbindung der allgemeinen Formel XIX

$$CH_3-X \quad (XIX)$$

in der

X eine nukleophile Austrittsgruppe wie ein Halogenatom oder eine Sulfonyloxygruppe, z.B. ein Chlor-, Brom- oder Jodatom, eine Methansulfonyloxy-, p-Toluolsulfonyloxy- oder Methoxysulfonyloxygruppe, oder, falls mindestens einer der Reste $R_1$ bis $R_4$ eine Hydroxygruppe darstellt, X zusammen mit einem Wasserstoffatom des $CH_3$-Restes eine Diazogruppe oder auch, falls $R_6$ ein Wasserstoffatom oder $R_4$ eine Amino- oder Methylaminogruppe darstellen, ein Sauerstoffatom bedeuten.

Die Umsetzung wird zweckmässigerweise in einem Lösungsmittel oder Lösungsmittelgemisch wie Diethylether, Methanol, Aceton, Tetrahydrofuran, Dioxan, Acetonitril, Pyridin oder Dimethylformamid gegebenenfalls in Gegenwart einer Base wie Kaliumcarbonat, Kaliumhydroxid, Kalium-tert.butylat oder Natriumhydrid bei Temperaturen zwischen 0 und 150 °C, vorzugsweise jedoch bei Temperaturen zwischen 20 und 120 °C, durchgeführt.

Bedeutet B eine $-CH_2-$ oder $-CO-$Gruppe und X eine nukleophile Austrittsgruppe, so wird die Umsetzung vorzugsweise mit einem Methylierungsmittel wie Methyljodid oder Dimethylsulfat in Gegenwart eines säurebindenden Mittels bei Temperaturen zwischen 20 und 80 °C durchgeführt. Die Umsetzung kann jedoch auch ohne Lösungsmittel durchgeführt werden.

Bedeutet X zusammen mit einem Wasserstoffatom der Methylgruppe eine Diazogruppe, so wird zur Methylierung einer Hydroxygruppe die Umsetzung mit Diazomethan bei Temperaturen zwischen 0 und 30 °C durchgeführt, oder ein Sauerstoffatom, so wird zur Methylierung des Stickstoffatoms die Umsetzung mit Formaldehyd in Gegenwart eines Reduktionsmittels bei Temperaturen zwischen 0 und 120 °C, z.B. mit Ameisensäure bei Temperaturen zwischen 80 und 110 °C oder mit Natriumcyanborhydrid bei Raumtemperatur und einem pH-Wert von 6–7, durchgeführt.

n) Zur Herstellung einer Verbindung der allgemeinen Formel I, in der A die $-COCO-$Gruppe und G eine n-Alkylengruppe mit 2 bis 5 Kohlenstoffatomen darstellen:

Oxidation einer Verbindung der allgemeinen Formel XX

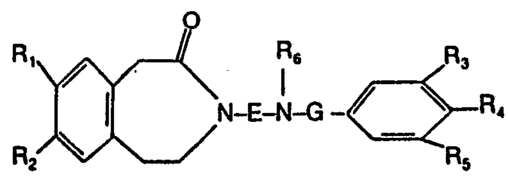

(XX)

in der

$R_1$ bis $R_6$, E und G wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 0 und 100 °C, vorzugsweise bei Temperaturen zwischen 20 und 80 °C, durchgeführt.

o) Zur Herstellung einer Verbindung der allgemeinen Formel I, in der A die $-CH_2-CH_2-$Gruppe, B eine $-CO-$Gruppe und G eine n-Alkylengruppe mit 2 bis 5 Kohlenstoffatomen darstellen:

Oxidation eines Benzazepins der allgemeinen Formel XXI

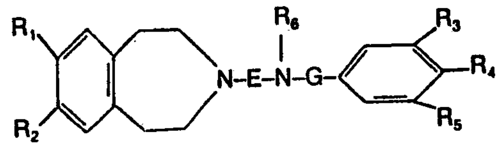

(XXI)

in der

$R_1$ bis $R_6$, E und G wie eingangs definiert sind, mit Ruthenium-tetroxid.

Die Oxidation wird mit Ruthenium-tetroxid in einem geeigneten Lösungsmittel oder Lösungsmittelgemisch wie Chloroform/Wasser, Methylenchlorid/Wasser oder Tetrachlorkohlenstoff/Wasser bei Temperaturen zwischen 0 und 100 °C, vorzugsweise bei Temperaturen zwischen 20 und 50 °C, durchgeführt. Die Umsetzung wird jedoch vorzugsweise in einem Zweiphasensystem wie Methylenchlorid/Wasser, Chloroform/Wasser oder Tetrachlorkohlenstoff/Wasser mit einer katalytischen Menge an Rutheniumdioxid in Gegenwart eines geeigneten Oxidationsmittels wie Natriumperjodat, welches Rutheniumdioxid in situ erzeugt, durchgeführt.

p) Zur Herstellung einer Verbindung der allgemeinen Formel I, in der A die $-CH_2-CH_2$-Gruppe und B die $-CO$-Gruppe darstellen:

Oxidation einer Verbindung der allgemeinen Formel XXII

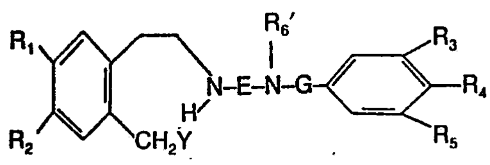
(XXII)

in der

$R_1$ bis $R_5$, G und E wie eingangs definiert sind,

$R_6'$ eine Methylengruppe und

Y eine für den Ringschluss geeignete Gruppe darstellt.

Für Y kommt beispielsweise die Carboxy- oder Nitrilgruppe, eine Estergruppe wie die Methoxycarbonyl-, Ethoxycarbonyl-, n-Propoxycarbonyl- oder Benzyloxycarbonylgruppe, eine Thioestergruppe wie die Ethylthiocarbonyl-, Phenylthiocarbonyl- oder Pyridylthiocarbonylgruppe, eine Acyloxycarbonylgruppe wie die Acetoxycarbonyl- oder Trifluoroacetylcarbonylgruppe oder eine Amidgruppe wie Aminocarbonyl-, Methylaminocarbonyl-, Dimethylaminocarbonyl- oder Phenylaminocarbonylgruppe in Betracht.

Die Umsetzung wird gegebenenfalls in Gegenwart eines geeigneten Kondensationsmittels und gegebenenfalls in einem Druckgefäss in einem Lösungsmittel wie Xylol, Dimethylglykoläther, Tetralin oder Sulfolan bei erhöhten Temperaturen, z.B. bei Temperaturen zwischen 100 und 250 °C, vorzugsweise jedoch bei Temperaturen zwischen 140 und 180 °C, durchgeführt. Die Umsetzung kann jedoch auch ohne Lösungsmittel durchgeführt werden.

Als geeignete Kondensationsmittel kommen beispielsweise N,N'-Dicyclohexyl-carbodiimid, Thionylchlorid, ein Phosphat wie Diethylchlorphosphonat oder Bis(O-nitrophenyl)-phenylphosphonat, ein Phosphoran wie (2,2,2-Trifluoroethoxy)-triphenylphosphoran, ein N-Alkyl-pyridiniumsalz wie N-Methyl-2-chlorpyridinium-jodid oder N-Methyl-2-fluor-pyridinium-tosylat, N,N'-Dicyclohexyl-carbodiimid/4-Dimethylamino-pyridin, Chlorsulfonylisocyanat oder N,N'-Carbonyldiimidazol in Betracht.

Desweiteren kann es von Vorteil sein, wenn gegebenenfalls vorhandene HO-, –HN- oder $H_2N$-Gruppen während der Umsetzung durch Schutzgruppen, z.B. durch Acetyl-, Benzoyl-, Ethoxycarbonyl- oder Benzylgruppen, geschützt werden.

Bedeutet Y in einer Verbindung der allgemeinen Formel XXII eine Nitril- oder Amidgruppe, so wird die Umsetzung bevorzugt in der Weise durchgeführt, dass eine entsprechende Verbindung mittels alkalischer oder saurer Hydrolyse, z.B. mittels Methanol/Salzsäure oder Ethanol/Natronlauge jeweils bei der Siedetemperatur des Reaktionsgemisches, in eine entsprechende Carboxyverbindung übergeführt wird, welche anschliessend cyclisiert wird.

Die gegebenenfalls während der Umsetzung verwendeten Schutzreste werden anschliessend wieder abgespalten, z.B. Acylreste vorzugsweise hydrolytisch in Gegenwart einer Säure oder Base bzw. Benzylreste vorzugsweise hydrogenolytisch, z.B. mit Wasserstoff in Gegenwart eines Hydrierungskatalysators wie Palladium/Kohle oder Platin.

Die so erhaltenen Verbindungen der allgemeinen Formel I lassen sich ferner in ihre Säureadditionssalze, insbesondere in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Citronensäure, Weinsäure, Bersteinsäure, Maleinsäure oder Fumarsäure in Betracht.

Die als Ausgangsstoffe verwendeten Verbindungen der allgemeinen Formeln II bis XXII sind teilweise literaturbekannt bzw. man erhält sie nach an sich bekannten Verfahren.

So erhält man beispielsweise eine Ausgangsverbindung der allgemeinen Formeln II und VIII durch Umsetzung eines entsprechenden Benzazepins mit einer entsprechenden Halogenverbindung und gegebenenfalls durch anschliessende Umsetzung mit einem entsprechenden Amin. Das hierfür erforderliche Benzazepin der allgemeinen Formel IV erhält man durch Cyclisierung einer entsprechenden Verbindung, z.B. durch Cyclisierung einer Verbindung der allgemeinen Formel XXIII

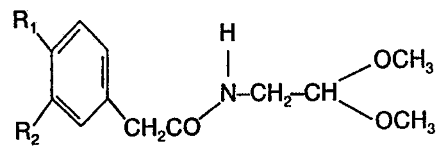
(XXIII)

gegebenenfalls anschliessender katalytischer Hydrierung und/oder Reduktion der Carbonylgruppe beispielsweise mit Natriumborhydrid/Eisessig (siehe EP-Al-0 007 070) und/oder Oxidation, z.B. mit Selendioxid.

Eine als Ausgangsverbindung verwendete Verbindung der allgemeinen Formel VI erhält man beispielsweise durch Umsetzung eines entsprechenden Benzazepins mit einem entsprechenden Halogenacetal und anschliessender Hydrolyse.

Eine als Ausgangsstoffe verwendete Verbindung der allgemeinen Formel XV erhält man durch Reduktion einer entsprechenden Nitroverbindung, welche man ihrerseits durch Acylierung bzw. Alkylierung eines entsprechenden Amins erhält.

Eine als Ausgangsstoff verwendete Verbindung der allgemeinen Formeln X bis XIII, XVI bis XVIII, XX und XXI erhält man vorzugsweise durch Umsetzung einer entsprechenden Halogenverbindung mit einem entsprechenden Amin und gegebenenfalls anschliessende Abspaltung von Schutzresten, die zum Schutze von Hydroxygruppen verwendet werden.

Eine als Ausgangsstoff verwendete Verbindung der allgemeinen Formel XXII erhält man beispielsweise durch Chlormethylierung einer Verbindung der allgemeinen Formel XXIV

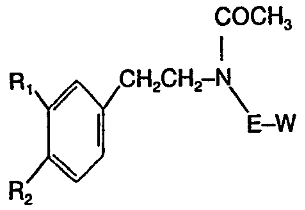

(XXIV)

in der

$R_1$, $R_2$ und E wie eingangs definiert sind und W eine geschützte Hydroxygruppe, z.B. die Acetoxy-, Benzoyloxy- oder 4-Nitrobenzoyloxygruppe, darstellt, weitere Umsetzung mit einem Alkalicyanid, Überführung des Restes W in eine geeignete Austrittsgruppe wie in die des Chlor-, Brom- oder Jodatoms, der Methylsulfonyloxy- oder 4-Methylphenylsulfonyloxygruppe und anschliessende Umsetzung mit einem Amin der allgemeinen Formel VII

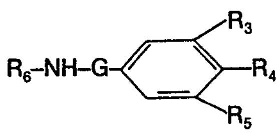

(VII)

in der

$R_3$ bis $R_6$ und G wie eingangs definiert sind, und gegebenenfalls anschliessende Hydrolyse und/oder Veresterung oder Amidierung.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere bei geringen Nebenwirkungen, z.B. einer geringen antimuskarinischen, eine lang anhaltende herzfrequenzsenkende Wirkung sowie eine Herabsetzung des $O_2$-Bedarfs des Herzens. Beispielsweise wurden die Verbindungen

A = 1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid,

B = 1-(7,8-Dimethoxy-1,3,4,5-tetrahydro-2H-3-benzazepin-2-thion-3-yl)-N-[N-methyl-N-(2-(3,4-dimethoxy-phenyl)-ethyl)-amino]-propan,

C = 1-(7,8-Dimethoxy-1,3,4,5-tetrahydro-2H-3-benzazepin-2-on-3-yl)-e-[N-methyl-N-(2-hydroxy-2-(3,4-dimethoxy-phenyl)-ethyl)-amino]-propan,

D = 1-(1-Hydroxy-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan,

E = 1-(7,8-Dimethoxy-1,3,4,5-tetrahydro-2H-3-benzazepin-2-on-3-yl)-3-[N-methyl-N-(2-(4-amino-3-nitro-phenyl)-ethyl)-amino]-propan-hydrochlorid,

F = 1-(7,8-Dimethoxy-1,3,4,5-tetrahydro-2H-3-benzazepin-2-on-3-yl)-3-[N-methyl-N-(4-(4-dimethylamino-phenyl)-butyl)-amino]-propan-hydrobromid,

G = 1-(7,8-Dimethoxy-1,3,4,5-tetrahydro-2H-3-benzazepin-2-on-3-yl)-3-[N-methyl-N-(2-phenyl-ethyl)-amino]-propan-dihydrochlorid,

H = 1-(7,8-Dimethoxy-1,3,4,5-tetrahydro-2H-3-benzazepin-2-on-3-yl)-3-[N-methyl-N-(5-(3,4-dimethoxy-phenyl)-pentyl)-amino]-propanhydrochlorid und

I = 1-(7,8-Dimethoxy-1,3,4,5-tetrahydro-2H-3-benzazepin-2-on-3-yl)-3-[N-methyl-N-(3-(4-amino-3,5-dichlor-phenyl)-propyl)-amino]-propan

im Vergleich zu

K = 1-(7,8-Dimethoxy-1,3,4,5-tetrahydro-5H-2-benzazepin-1-on-2-yl)-3-[N-methyl-N-(2-(3,4-dimethoxy-phenyl)-ethyl)-amino]-propanhydrochlorid

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250–300 g untersucht. Hierzu wurden die Ratten mit Pentobarbital (50 mg/kg i.p. und 20 mg/kg s.c.) narkotisiert. Die zu untersuchenden Substanzen wurden in wässriger Lösung in die Vena jugularis injiziert (0.1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minute (S/min).

Die nachfolgende Tabelle enthält die gefundenen Werte:

| Substanz | Dosis [mg/kg] | Herzfrequenzsenkung, gemessen 20 Minuten nach Substanzapplikation [S/min] |
|---|---|---|
| A | 5,0 | −183 |
| | 2,5 | −85 |
| | 1,0 | −51 |
| B | 5,0 | −255 |
| | 2,5 | −134 |
| | 1,0 | −73 |
| C | 5,0 | −173 |
| | 2,5 | −137 |
| D | 5,0 | −117 |
| | 2,5 | −73 |
| | 1,0 | −83 |
| E | 5,0 | −130 |
| F | 5,0 | −123 |
| | 2,5 | −91 |
| | 1,0 | −94 |
| G | 5,0 | −135 |
| | 2,5 | −110 |
| | 1,0 | −80 |
| H | 5,0 | −75 |
| I | 5,0 | −175 |
| K | 5,0 | −18 |

Die erfindungsgemäss hergestellten Verbindungen weisen in therapeutischen Dosen keinerlei toxische Nebenwirkungen auf. So konnten beispielsweise bei einer intravenösen Applikation der Substanzen A und D auch in einer hohen Dosis von 20 mg/kg an Mäusen keine toxischen Nebenwirkungen beobachtet werden.

Aufgrund ihrer pharmakologischen Eigenschaften eignen sich die erfindungsgemässen Verbindungen zur Behandlung von Sinustachykardien verschiedener Genese und zur Prophylaxe und Therapie ischämischer Herzerkrankungen.

Die zur Erzielung einer entsprechenden Wirkung erforderliche Dosierung beträgt zweckmässigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemässen Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Cellulose, Magnesiumstearat, Polyvinylpyrrolidon, Citronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern:

Herstellung der Ausgangsverbindung:
Beispiel A
1-(7,8-Dimethoxy-1,3,4,5-tetrahydro-2H-3-benzazepin-2-on-3-yl)-3-N-methyl-amino-propan-hydrochlorid

a) 1-(7,8-Dimethoxy-1,3-dihydro-2H-3-benzazepin-2-on-3-yl)-3-N-methyl-N-benzyl-amino-propanhydrochlorid

Hergestellt analog Beispiel 1b durch Umsetzung von
1-(7,8-Dimethoxy-1,3-dihydro-2H-3-benzazepin-2-on-3-yl)-3-chlor-propan
mit N-Methyl-benzylamin.
Ausbeute: 92,1% der Theorie,
Schmelzpunkt: 208–209 °C.

b) 1-(7,8-Dimethoxy-1,3,4,5-tetrahydro-2H-3-benzazepin-2-on-3-yl)-3-N-methyl-amino-propan-hydrochlorid

Hergestellt analog Beispiel 4 durch katalytische Hydrierung von
1-(7,8-Dimethoxy-1,3-dihydro-2H-3-benzazepin-2-on-3-yl)-3-N-Methyl-N-benzyl-amino-propan.
Ausbeute: 87% der Theorie,
Schmelzpunkt: 110 °C (Zers.).

Herstellung der Endprodukte:
Beispiel 1
1-(7,8-Dimethoxy-1,3,4,5-tetrahydro-2H-3-benzazepin-2-on-3-yl)-2-[N-methyl-N-(3-(3,4-dimethoxy-phenyl)-propyl)-amino]-propan-hydrochlorid

a) 1-(7,8-Dimethoxy-1,3,4,5-tetrahydro-2H-3-benzazepin-2-on-3-yl)-3-chlor-propan

1,1 g (0,005 Mol) 7,8-Dimethoxy-1,3,4,5-tetrahydro-2H-3-benzazepin-2-on werden in 15 ml absolutem Dimethylsulfoxid suspendiert und unter Rühren mit 0,67 g (0,006 Mol) Kalium-tert.butylat versetzt. Nach 10 Minuten wird die erhaltene Suspension unter Kühlung mit Eiswasser zu 0,64 ml (0,006 Mol) 1-Brom-3-chlor-propan in 10 ml Dimethylsulfoxid getropft. Nach einer Stunde giesst man auf Wasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.
Ausbeute: 0,75 g (50,0% der Theorie),
Schmelzpunkt: 84–85 °C.

b) 1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid

5,55 g (0,0186 Mol)
1-(7,8-Dimethoxy-1,3,4,5-tetrahydro-2H-3-benzazepin-2-on-3-yl)-3-chlor-propan,
2,6 ml (0, 0886 Mol) Triethylamin und 3,9 g (0,0186 Mol) N-Methyl-3-(3,4-dimethoxy-phenyl)-propylamin werden 4 Stunden auf 85 °C erhitzt, abgekühlt und in Methylenchlorid/Wasser gelöst. Die organische Phase wird abgetrennt, noch einmal mit Wasser extrahiert, über Magnesiumsulfat getrocknet, im Vakuum eingeengt und der erhaltene Rückstand über Kieselgel mit Methylenchlorid +3% Ethanol als Elutionsmittel gereinigt. Der erhaltene ölige Rückstand wird in Aceton gelöst und mit etherischer Salzsäure das Hydrochlorid gefällt.
Ausbeute: 3,45 g (36,6% der Theorie),
Schmelzpunkt: 220–221 °C.

Beispiel 2
1-(7,8-Dimethoxy-1,3,4,5-tetrahydro-2H-3-benzazepin-2-on-3-yl)-3-[N-methyl-N-(2-phenyl-ethyl)-amino]-propan-dihydrochlorid

Hergestellt analog Beispiel 1b durch Umsetzung von
1-(7,8-Dimethoxy-1,3,4,5-tetrahydro-2H-3-benzazepin-2-on-3-yl)-3-chlor-propan
mit N-Methyl-2-phenyl-ethylamin.
Ausbeute: 43,2% der Theorie,
Schmelzpunkt: 165 °C (Zers.).

Beispiel 3
1-(7,8-Dimethoxy-1,3-dihydro-2H-3-benzazepin-2-on-3-yl)-3-[N-methyl-N-(5-(3,4-dimethoxy-phenyl)-pentyl)-amino]-propan-hydrochlorid

a) 1-(7,8-Dimethoxy-1,3-dihydro-2H-3-benzazepin-2-on-3-yl)-3-chlor-propan

Hergestellt analog Beispiel 1a durch Umsetzung von

7,8-Dimethoxy-1,3-dihydro-2H-3-benzazepin-2-on mit 1-Brom-3-chlor-propan.

Ausbeute: 87,3% der Theorie,
Schmelzpunkt: 101–103 °C.

b) 1-(7,8-Dimethoxy-1,3-dihydro-2H-benzazepin-2-on-3-yl)-3-[N-methyl-N-(5-(3,4-dimethoxy-phenyl)-pentyl)-amino]-propan-hydrochlorid

Hergestellt analog Beispiel 1b durch Umsetzung von 1-(7,8-Dimethoxy-1,3-dihydro-2H-3-benzazepin-2-on-3-yl)-3-chlor-propan mit N-Methyl-N-(5-(3,4-dimethoxy-phenyl)-pentyl)-amin.

Ausbeute: 67,3% der Theorie,
Schmelzpunkt: 158–160 °C.

Beispiel 4

1-(7,8-Dimethoxy-1,3,4,5-tetrahydro-2H-3-benzazepin-2-on-3-yl)-3-[N-methyl-N-(5-(3,4-dimethoxy-phenyl)-pentyl)-amino]-propan-hydrochlorid

3,23 g (0,0065 Mol) 1-(7,8-Dimethoxy-1,3-dihydro-2H-3-benzazepin-2-on-3-yl)-3-[N-methyl-N-(5-(3,4-dimethoxy-phenyl)-pentyl)-amino]-propan, gelöst in 30 ml Essigsäure, werden bei Raumtemperatur und einem Wasserstoffdruck von 5 bar in Anwesenheit von 10%iger Palladium/Kohle 3,5 Stunden hydriert. Der Katalysator wird abfiltriert, das Filtrat im Vakuum eingeengt und der Rückstand in Methylenchlorid/15%iger Kaliumcarbonat-Lösung aufgenommen. Die organische Phase wird abgetrennt, über Mangnesiumsulfat getrocknet, im Vakuum einrotiert und der erhaltene Rückstand über Kieselgel mit Methylenchlorid +4% Ethanol als Elutionsmittel gereinigt. Der erhaltene Rückstand wird in Aceton gelöst und mit etherischer Salzsäure das Hydrochlorid gefällt.

Ausbeute: 2,1 g (60,3% der Theorie),
Schmelzpunkt: 165–166 °C.

Beispiel 5

1-(7,8-Dimethoxy-1,3,4,5-tetrahydro-2H-3-benzazepin-2-thion-3-yl)-3-[N-methyl-N-(2-(3,4-dimethoxy-phenyl)-ethyl)-amino]-propan

2,28 g (0,005 Mol) 1-(7,8-Dimethoxy-1,3,4,5-tetrahydro-2H-3-benzazepin-2-on3-yl)-3-[N-methyl-N-(2-(3,4-dimethoxy-phenyl)-ethyl)-amino]-propan werden in 10 ml absolutem Toluol gelöst und mit 1,0 g (0,0025 Mol) 2,4-Bis-(4-methoxy-phenyl)-1,3-dithia-2,4-diphosphetane-2,4-disulfid 50 Minuten am Rückfluss gekocht. Nach Einrotieren des Lösungsmittels im Vakuum wird der erhaltene Rückstand über Aluminiumoxid mit Methylenchlorid +2% Ethanol als Elutionsmittel gereinigt.

Ausbeute: 1,45 g (61,4% der Theorie),

$C_{26}H_{36}N_2O_4S$ (472,6)

| | | | | |
|---|---|---|---|---|
| Ber.: | C 66,07 | H 7,68 | N 5,93 | S 6,78%; |
| Gef.: | C 66,10 | H 7,71 | N 5,56 | S 6,76%. |

Beispiel 6

1-(7,8-Dimethoxy-1,3,4,5-tetrahydro-2H-3-benzazepin-2-on-3-yl)-3-[N-methyl-N-(2-hydroxy)-2-(3,4-dimethoxy-phenyl)-ethyl)-amino]-propan

a) 1-(7,8-Dimethoxy-1,3,4,5-tetrahydro-2H-3-benzazepin-2-on-3-yl)-3-[N-methyl-N-(3,4-dimethoxy-benzoyl)-methyl)-amino]-propan-hydrochlorid.

Hergestellt analog Beispiel 1b durch Umsetzung von 1-(7,8-Dimethoxy-1,3,4,5-tetrahydro-2H-3-benzazepin-2-on-3-yl)-3-[N-methyl-amino-propan mit α-Brom-3,4-dimethoxy-acetophenon.

Ausbeute: 1,29 g (77,0% der Theorie),
Schmelzpunkt: 190 °C.

b) 0,92 g (0,002 Mol) 1-(7,8-Dimethoxy-1,3,4,5-tetrahydro-2H-3-benzazepin-2-on-3-yl)-3-[N-methyl-N-(3,4-dimethoxy-benzoyl)-methyl)-amino]-propan werden in 6 ml Ethanol gelöst, mit 0,38 g (0,01 Mol) Natriumborhydrid versetzt und 2 Stunden bei 25 °C gerührt. Nach Einengen des Lösungsmittels im Vakuum wird in Methylenchlorid gelöst, mit Wasser extrahiert, über Magnesiumsulfat getrocknet, im Vakuum eingeengt und der erhaltene Rückstand über Aluminiumoxid mit Methylenchlorid +1% Ethanol als Elutionsmittel gereinigt.

Ausbeute: 0,5 g (54% der Theorie),
IR-Spektrum (Methylenchlorid): 1655 $cm^{-1}$ (CO)

$C_{26}H_{36}N_2O_6$ (472,6)

| | | | |
|---|---|---|---|
| Ber.: | C 66,08 | H 7,68 | N 5,93%; |
| Gef.: | C 66,01 | H 7,62 | N 5,80%. |

Beispiel 7

1-(1-Hydroxy-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan

2,35 (5 mMol) 1-(7,8-Dimethoxy-1,3,4,5-tetrahydro-2H-3-benzazepin-1,2-dion-3-yl)-3-[N-methyl-N-(2-(3,4-dimethoxy-phenyl)-ethyl)-amino]-propan werden in 100 ml Methanol und 5 ml Wasser gelöst und unter Rühren portionenweise mit 0,2 g Natriumborhydrid versetzt. Nach Beendigung der Zugabe wird 15 Minuten weitergerührt, mit 10 ml 2 n Salzsäure versetzt, mit methanolischem Ammoniak alkalisch gestellt, mit Bleicherde entfärbt und nach Filtration im Vakuum eingeengt. Der Rückstand wird in Methylenchlorid gelöst, ungelöste Salze abfiltriert und das Filtrat eingeengt.

Ausbeute: 1,65 g (69,9% der Theorie), viskoses Öl.

IR-Spektrum (Methylenchlorid): 3400 $cm^{-1}$ (OH), 2840 $cm^{-1}$ (Methoxyl), 2800 $cm^{-1}$ (N-Alkyl), 1660 $cm^{-1}$ (CO)

$C_{26}H_{36}N_2O_6$ (472,59)

| | | | |
|---|---|---|---|
| Ber.: | C 66,08 | H 7,68 | N 5,93%; |
| Gef.: | C 65,87 | H 7,75 | N 5,73%. |

Beispiel 8

1-(7,8-Dimethoxy-4,5-dihydro-3H-1,3-benzodiazepin-3-yl)-3-[N-methyl-N-(2-(3,4-dimethoxy-phenyl)-ethyl)-amino]-propan-dihydrochlorid

4,3 g (10 mMol) N-[2-(2-Amino-4,5-dimethoxy-phenyl)-ethyl]-N'-methyl-N'-(2-(3,4-dimethoxy-phenyl)-ethyl)-1,3-diamino-propan werden in 30 ml Orthoameisensäureester 18 Stunden unter Rückfluss erhitzt, im Vakuum eingeengt und der Rückstand über Kieselgel mit Methylenchlorid +3% Methanol als Elutionsmittel gereinigt. Der erhaltene Rückstand wird in Aceton gelöst und durch Zugabe von etherischer Salzsäure das Dihydrochlorid gefällt.

Ausbeute: 1,6 g (31,1% der Theorie),
Schmelzpunkt: 232–234 °C.

Beispiel 9

1-(7,8-Dimethoxy-1,3,4,5-tetrahydro-2H-3-benzazepin-2-on-3-yl)-3-[N-methyl-N-(4-amino-3-nitro-phenyl)-ethyl)-amino]-propan-hydrochlorid

1,0 g (1,75 mMol)
1-(7,8-Dimethoxy-1,3,4,5-tetrahydro-2H-3-benzazepin-2-on-3-yl)-3-[N-methyl-N-(2-(4-acetamino-3-nitrophenyl)-ethyl)-amino]-propan
wird in 50 ml methanolischer Salzsäure 2 Stunden auf 45–50 °C erwärmt. Die Reaktionslösung wird eingeengt, in Methylenchlorid gelöst, mit gesättigter Natriumhydrogencarbonat-Lösung gewaschen, über Magnesiumsulfat getrocknet und das Lösungsmittel im Vakuum abdestilliert. Nach Reinigung des Rückstandes über Kieselgel mit Methylenchlorid und 5% Ethanol als Elutionsmittel wird aus Aceton durch Zugabe von etherischer Salzsäure das Hydrochlorid gefällt.

Ausbeute: 0,4 g (43,1% der Theorie).,
Schmelzpunkt: 207–208 °C (Zers.).

Beispiel 10

1-(1-Oximino-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan-hydrochlorid

3,6 g (6,8 mMol)
1-(7,8-Dimethoxy-1,3,4,5-tetrahydro-2H-3-benzazepin-1,2-dion-3-yl)-3-[N-methyl-N-(2-(3,4-dimethoxy-phenyl)-ethyl)-amino]-propan,
0,57 g Hydroxylamin-hydrochlorid und 0,87 g Natriumcarbonat werden in 100 ml Ethanol 8 Stunden unter Rückfluss erhitzt. Das Lösungsmittel wird im Vakuum abdestilliert, der Rückstand in Wasser/Methylenchlorid gelöst, die organische Phase abgetrennt, über Magnesiumsulfat getrocknet und eingeengt. Der Rückstand wird über Kieselgel mit Methylenchlorid und 5% Methanol als Elutionsmittel gereinigt. Der erhaltene Rückstand wird in Aceton gelöst und durch Zugabe von etherischer Salzsäure das Hydrochlorid gefällt.

Ausbeute: 2,4 g (67,6% der Theorie),
Schmelzpunkt: 156–158 °C.

Beispiel 11

1-(1-Amino-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan-dihydrochlorid

1,6 g (3,3 mMol)
1-(1-Oximino-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan
werden in 150 ml Ethanol mit 1,5 ml 98%igem Hydrazinhydrat und 1 g Raney-Nickel versetzt und 7 Stunden unter Rückfluss erhitzt. Zur Vervollständigung der Reaktion werden weitere 1,5 ml 98%iges Hydrazinhydrat und 1 g Raney-Nickel zugesetzt und nochmals 3 Stunden unter Rückfluss erhitzt. Der Katalysator wird abgesaugt, das Lösungsmittel im Vakuum abdestilliert und der Rückstand über Kieselgel mit Methylenchlorid und 10% Methanol gereinigt. Aus einer Aceton-Lösung wird durch Zusatz von etherischer Salzsäure das Dihydrochlorid gefällt.

Ausbeute: 0,8 g (44,6% der Theorie),
Schmelzpunkt: 232–234 °C,
m/e = 471.

Beispiel 12

1-(7,8-Dimethoxy-1,3,4,5-tetrahydro-2H-3-benzazepin-2-on-3-yl)-3-[N-methyl-N-(3-(4-dimethylamino-phenyl)-propyl)-amino]-propan-dihydrochlorid

5,7 g (19,4 mMol)
1-(7,8-Dimethoxy-phenyl-1,3,4,5-tetrahydro-2H-3-benzazepin-2-on-3-yl)-3-N-methyl-amino-propan
und 4,7 g (19,4 mMol) 3-(4-Dimethylamino-phenyl)-propylbromid werden nach Zugabe von 3,3 ml Ethyldiisopropylamin 1½ Stunden auf 130 °C erhitzt. Das Reaktionsgemisch wird in Chloroform und 25%iger Natronlauge gelöst, die organische Phase abgetrennt, mit Wasser gewaschen, über Magnesiumsulfat getrocknet und eingeengt. Nach Reinigung des erhaltenen Rückstandes über Kieselgel mit Methylenchlorid und 5% Methanol als Elutionsmittel wird aus Aceton mit ätherischer Salzsäure das Dihydrochlorid gefällt.

Ausbeute: 0,6 g (5,9% der Theorie),
Schmelzpunkt: 191–192 °C (Zers.).

Beispiel 13

1-(7,8-Dimethoxy-1,3,4,5-tetrahydro-2H-3-benzazepin-2-on-3-yl)-3-[N-methyl-N-(4-(4-dimethylamino-phenyl)-butyl)-amino]-propan-hydrochlorid

Hergestellt analog Beispiel 12 aus
1-(7,8-Dimethoxy-phenyl-1,3,4,5-tetrahydro-2H-3-benzazepin-2-on-3-yl)-3-N-methyl-amino-propan
und 4-(4-Dimethylamino-phenyl)-butylbromid.

Ausbeute: 10,3% der Theorie,
Schmelzpunkt: 116–118 °C.

Beispiel 14

1-(7,8-Dimethoxy-1,3,4,5-tetrahydro-2H-3-benzazepin-2-on-3-yl)-3-[N-methyl-N-(4-(4-amino-3,5-dibrom-phenyl)-butyl)-amino]-propan-hydrobromid

1,1 g (3,6 mMol)
1-(7,8-Dimethoxy-phenyl-1,3,4,5-tetrahydro-2H-3-benzazepin-2-on-3-yl)-3-N-methyl-amino-propan
und 1,4 g (3,6 mMol) 4-(4-Amino-3,5-dibromphenyl)-butylbromid werden in 3 ml Ethyldiisopropylamin 2 Stunden auf 130 °C erhitzt. Anschliessend wird im Vakuum das überschüssige Amin abdestilliert und der erhaltene Rückstand über Kieselgel mit Methylenchlorid und 2% Ethanol als Elutionsmittel gereinigt. Die Fraktionen werden im

Vakuum eingeengt, der Rückstand mit Aceton verrieben und der entstandene Niederschlag abgesaugt.

Ausbeute: 0,6 g (27,9% der Theorie),
Schmelzpunkt: 159–161 °C.

Beispiel 15

1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid

2,6 g (5 mMol)
N-[3-[N'-Methyl-N'-(3-(3,4-dimethoxy-phenyl)-propyl)-amino]-propyl]-2-(2-carboxymethyl-4,5-dimethoxyphenyl)-ethylamin-Natriumsalz
werden in 30 ml Sulfolan 2 Stunden auf 200 °C erhitzt. Nach dem Erkalten wird mit 300 ml Wasser verdünnt und 3 mal mit Methylenchlorid extrahiert. Die organischen Extrakte werden 2 mal mit Wasser gewaschen, über Magnesiumsulfat getrocknet und das Lösungsmittel im Vakuum eingeengt. Der Rückstand wird in Aceton gelöst und durch Zugabe von methanolischer Salzsäure das Hydrochlorid gefällt.

Ausbeute: 1,8 g (71% der Theorie),
Schmelzpunkt: 220–221 °C.

Beispiel 16

1-(7,8-Dimethoxy-1,3,4,5-tetrahydro-2H-3-benzazepin-1,2-dion-3-yl)-3-[N-methyl-N-(3-(3,4-dimethoxy-phenyl)-propyl)-amino]-propan-hydrochlorid

1,7 g (0,0154 Mol) Selendioxid werden bei 70 °C zu 70 ml 1,4-Dioxan und 2,8 ml Wasser gegeben. Nach 15 Minuten gibt man 1,4 g Celite und 6,9 g (0,0147 Mol)
1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan
zu und kocht 40 Stunden am Rückfluss. Nach dem Abkühlen werden die ungelösten Bestandteile abgesaugt, das Filtrat einrotiert und der erhaltene Rückstand über Kieselgel mit Methylenchlorid +4% Ethanol als Elutionsmittel gereinigt. Das Produkt wird in Aceton gelöst und mit etherischer Salzsäure das Hydrochlorid gefällt.

Ausbeute: 2,36 g (29,2% der Theorie),
Schmelzpunkt: 189–192 °C.

Beispiel 17

1-(7,8-Dimethoxy-1,3,4,5-tetrahydro-2H-3-benzazepin-2-on-3-yl)-3-[N-methyl-N-(2-(4-amino-3,5-dichlor-phenyl)-2-hydroxy-ethyl)-amino]-propan

Hergestellt aus
1-(7,8-Dimethoxy-1,3,4,5-tetrahydro-2H-3-benzazepin-2-on-3-yl)-3-[N-methyl-N-(4-amino-3,5-dichlor-benzoyl-methyl)-amino]-propan
und Natriumborhydrid analog Beispiel 6.

Ausbeute: 71,6% der Theorie,
Schmelzpunkt: 122–126 °C.
IR-Spektrum (Methylenchlorid): 3400 $cm^{-1}$, 3495 $cm^{-1}$ ($NH_2$), 1650 $cm^{-1}$ (CO);
UV-Spektrum (Ethanol): 240 nm (0,14), 290 nm (0,06), 314 nm (Schulter; 0,02).

Beispiel 18

1-(7,8-Dimethoxy-1,3,4,5-tetrahydro-2H-3-benzazepin-2-on-3-yl)-3-[N-methyl-N-(2-(3-amino-4-chlor-phenyl)-2-hydroxy-ethyl)-amino]-propan

Hergestellt aus
1-(7,8-Dimethoxy-1,3,4,5-tetrahydro-2H-3-benzazepin-2-on-3-yl)-3-[N-methyl-N-(3-amino-4-chlor-benzoyl-methyl)-amino]-propan
und Natriumborhydrid analog Beispiel 6.

Ausbeute: 55% der Theorie, Harz.
IR-Spektrum (Methylenchlorid): 3380 $cm^{-1}$, 3470 $cm^{-1}$ ($NH_2$), 1650 $cm^{-1}$ (Lactam-CO);
UV-Spektrum (Ethanol): 236 nm (0,13), 282–292 nm (0,055), 310 nm (Schulter; 0,02).

Beispiel 19

1-(7,8-Dimethoxy-1,3,4,5-tetrahydro-2H-3-benzazepin-2-on-3-yl)-[N-methyl-N-(2-(4-amino-3-chlor-5-fluor-phenyl)-2-hydroxy-ethyl)-amino]-propan

Hergestellt aus
1-(7,8-Dimethoxy-1,3,4,5-tetrahydro-2H-3-benzazepin-2-on-3-yl)-3-[N-methyl-N-(4-amino-3-chlor-5-fluor-benzoyl-methyl)-amino]-propan
und Natriumborhydrid analog Beispiel 6.

Ausbeute: 48% der Theorie, Schaum.
IR-Spektrum (Methylenchlorid): 3390 $cm^{-1}$, 3480 $cm^{-1}$ ($NH_2$), 1645 $cm^{-1}$ (Lactam-CO);
UV-Spektrum (Ethanol): 238 nm (0,18), 282–290 nm (0,07).

Beispiel 20

1-(7,8-Dimethoxy-1,3,4,5-tetrahydro-2H-3-benzazepin-2-on-3-yl)-3-[N-methyl-N-(2-(4-amino-3-chlor-5-methyl-phenyl)-2-hydroxy-ethyl)-amino]-propan

Hergestellt aus
1-(7,8-Dimethoxy-1,3,4,5-tetrahydro-2H-3-benzazepin-2-on-3-yl)-3-[N-methyl-N-(4-amino-3-chlor-5-methyl-benzoyl-methyl)-amino]-propan
und Natriumborhydrid analog Beispiel 6.

Ausbeute: 25% der Theorie, Schaum.
IR-Spektrum (Methylenchlorid): 3380 $cm^{-1}$, 3470 $cm^{-1}$ ($NH_2$), 1650 $cm^{-1}$ (Lactam-CO);
UV-Spektrum (Ethanol): 239 nm (0,15), 280–292 nm (0,05), 305 nm (Schulter; 0,01).

Analog den vorstehenden Beispielen lassen sich folgende Verbindungen herstellen:

1-(7,8-Dimethoxy-1,3,4,5-tetrahydro-2H-3-benzazepin-2-on-3-yl)-3-[N-methyl-N-(2-hydroxy-3-(3,4-dimethoxy-phenyl)-propyl)-amino]-propan, Öl
$C_{27}H_{38}N_2O_6$ (486,6)
Ber.: C 64,64 H 7,87 N 5,76%;
Gef.: C 64,61 H 7,95 N 5,74%.

1-(7,8-Dimethoxy-1,3,4,5-tetrahydro-2H-3-benzazepin-2-on-3-yl)-3-[N-methyl-N-(2-hydroxy-3-(4-amino-3,5-dichlor-phenyl)-propyl)-amino]-propan

1-(7,8-Dimethoxy-1,3,4,5-tetrahydro-2H-3-benzazepin-2-on-3-yl)-3-[N-methyl-N-(3-hydroxy-3-(4-amino-3,5-dichlor-phenyl)-propyl)-amino]-propan

1-(7,8-Dimethoxy-1,3,4,5-tetrahydro-2H-3-benzazepin-2-on-3-yl)-3-[N-methyl-N-(3-hydroxy-3-(3,4-dimethoxy-phenyl)-propyl)-amino]-propan

1-(7,8-Dimethoxy-1,3,4,5-tetrahydro-2H-3-benzazepin-2-on-3-yl)-3-[N-methyl-N-(3-(4-amino-3,5-dichlor-phenyl)-propyl)-amino]-propan
Schmelzpunkt: 92–93 °C

1-(1-Hydroxy-7,8-dimethoxy-1,3,4,5-tetrahydro-2H-3-benzazepin-2-on-3-yl)-3-[N-methyl-N-(3-(3,4-dimethoxy-phenyl)-propyl)-amino]-propan

1-(7,8-Dimethoxy-1,3,4,5-tetrahydro-2H-3-benzazepin-2-on-3-yl)-3-[N-3-(3,4-dimethoxy-phenyl)-propyl)-amino]-propan
IR-Spektrum (Methylenchlorid): 1645 $cm^{-1}$ (CO), Schmelzpunkt des Hydrochlorids: 158–159 °C

1-(7,8-Dimethoxy-1,3,4,5-tetrahydro-2H-3-benzazepin-2-on-3-yl)-3-[N-(3-(4-amino-3,5-dichlor-phenyl)-propyl)-amino]-propan

1-(7,8-Dimethoxy-1,3,4,5-tetrahydro-2H-3-benzazepin-2-on-3-yl)-3-[N-(2-hydroxy-3-(3,4-dimethoxy-phenyl)-propyl)-amino]-propan

Beispiel I

Tabletten zu 10 mg
1-(7,8-Dimethoxy-1,3,4,5-tetrahydro-2H-3-benzazepin-2-on-3-yl)-[N-methyl-N-(3-(3,4-dimethoxy-phenyl)-propyl)-amino]-propan-hydrochlorid

Zusammensetzung:
1 Tablette enthält:

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren:

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit Magnesiumstearat vermischt. Die fertige Mischung presst man auf einer Tablettenpresse mit Stempeln von 7 mm Durchmesser, die mit einer Teilkerbe versehen sind, zu Tabletten.

Tablettengewicht: 120 mg

Beispiel II

Dragées zu 5 mg
1-(7,8-Dimethoxy-1,3,4,5-tetrahydro-2H-3-benzazepin-2-on-3-yl)-3-[N-methyl-N-(3,4-dimethoxy-phenyl)-propyl)-amino]-propan-hydrochlorid

1 Dragéekern enthält:

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren:

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45 °C und schlägt das Granulat anschliessend durch dasselbe Sieb. Nach dem Zumischen von Magnesiumstearat werden auf einer Tablettiermaschine gewölbte Dragéekerne mit einem Durchmesser von 6 mm gepresst. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel III

Ampullen zu 5 mg
1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid

1 Ampulle enthält:

| | |
|---|---|
| Wirksubstanz | 5,0 mg |
| Sorbit | 50,0 mg |
| Wasser für Injektionszwecke ad | 2,0 ml |

Herstellungsverfahren:

In einem geeigneten Ansatzgefäss wird der Wirkstoff in Wasser für Injektionszwecke gelöst und die Lösung mit Sorbit isotonisch gestellt.

Nach Filtration über einen Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg
1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid

1 Zäpfchen enthält:

| | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.

Beispiel V

Tropfenlösung mit 10 mg
1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan-hydrochlorid

100 ml Lösung enthalten:

| | | |
|---|---|---|
| Wirksubstanz | | 0,2 g |
| Hydroxyäthylcellulose | | 0,15 g |
| Weinsäure | | 0,1 g |
| Sorbitlösung 70% Trockensubstanz | | 30,0 g |
| Glycerin | | 10,0 g |
| Benzoesäure | | 0,15 g |
| Dest. Wasser | ad | 100 ml |

Herstellungsverfahren:

Dest. Wasser wird auf 70 °C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschliessend wird zur Entlüftung des Saftes unter Rühren evakuiert.

**Patentansprüche**

1. Benzazepinderivate der allgemeinen Formel I

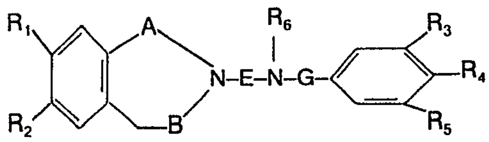 (I)

in der
A eine $-CH_2-CH_2-$ oder $-CH=CH-$Gruppe und
B die $-CS-$Gruppe oder auch, wenn
G eine n-Alkylengruppe mit 3 bis 5 Kohlenstoffatomen oder eine Methylen-n-hydroxyalkylengruppe mit 1 bis 3 Kohlenstoffatomen im Alkylenteil darstellt, wobei die Methylengruppe mit dem Stickstoffatom verknüpft ist, die $-CO-$Gruppe oder
A eine

OH N–OH NH₂
| ‖ |
$-N=CH-$, $-CH-CO-$, $-C-CO-$ oder $-CH-CO-$
x x x x

Gruppe

oder auch die $-COCO-$Gruppe, wenn
G eine n-Alkylengruppe mit 3 bis 5 Kohlenstoffatomen oder eine Methylen-n-hydroxyalkylengruppe mit 1 bis 3 Kohlenstoffatomen im Alkylenteil darstellt, wobei die Methylengruppe mit dem Stickstoffatom verknüpft ist, und
B die $-CH_2-$Gruppe, wobei das mit x gekennzeichnete Kohlenstoffatom jeweils mit dem Phenylkern verknüpft sein muss,
E eine n-Propylengruppe,
G eine n-Alkylengruppe mit 2 bis 5 Kohlenstoffatomen oder eine Methylen-n-hydroxyalkylengruppe mit 1 bis 3 Kohlenstoffatomen im Alkylenteil, wobei die Methylengruppe mit dem Stickstoffatom verknüpft ist,
$R_1$ und $R_2$ jeweils eine Methoxygruppe,
$R_3$ ein Wasserstoff-, Fluor-, Chlor- oder Bromatom, eine Methoxy- oder Nitrogruppe,
$R_4$ ein Wasserstoffatom, eine Methoxy-, Amino-, Methylamino- oder Dimethylaminogruppe,
$R_5$ ein Wasserstoff-, Chlor- oder Bromatom und
$R_6$ ein Wasserstoffatom oder eine Methylgruppe bedeuten, sowie
1-(7,8-Dimethoxy-1,3,4,5-tetrahydro-2H-3-benzazepin-2-on-3-yl)-3-[N-methyl-N-(2-phenylethyl)-amino]-propan und
1-(7,8-Dimethoxy-1,3,4,5-tetrahydro-2H-3-benzazepin-2-on-3-yl)-3-[N-methyl-N-(2-(4-amino-3-nitro-phenyl)-ethyl)-amino]-propan
und deren Säureadditionssalze.

2. Benzazepinderivate der allgemeinen Formel I gemäss Anspruch 1, in der
E wie im Anspruch 1 definiert ist,
A die $-CH_2CH_2-$Gruppe,
B die $-CO-$ oder $-CS-$Gruppe,
$R_1$ und $R_2$ jeweils eine Methoxygruppe,
$R_3$ ein Wasserstoff-, Fluor-, Chlor- oder Bromatom oder eine Methoxygruppe,
$R_4$ eine Methoxy-, Amino-, Methylamino- oder Dimethylaminogruppe,
$R_5$ ein Wasserstoff-, Chlor- oder Bromatom,
$R_6$ die Methylgruppe und
G eine n-Alkylengruppe mit 3 bis 5 Kohlenstoff-

OH
|
atomen oder die $-CH_2-CH-$Gruppe oder auch, wenn B die $-CS-$Gruppe darstellt, die $-CH_2CH_2-$Gruppe bedeuten, und deren Säureadditionssalze.

3. Benzazepinderivate der allgemeinen Formel I gemäss Anspruch 1, in der
E wie im Anspruch 1 definiert ist,
A die $-CH_2CH_2-$Gruppe und
B die $-CS-$Gruppe oder auch, wenn G eine n-Alkylengruppe mit 3 bis 5 Kohlenstoffatomen darstellt, die $-CO-$Gruppe oder

OH
|
A die $-CH-CO-$Gruppe und
B die $-CH_2-$Gruppe,
$R_1$ und $R_2$ je eine Methoxygruppe,
$R_3$ eine Methoxygruppe, ein Wasserstoff- oder Chloratom,
$R_4$ eine Methoxy-, Amino- oder Dimethylaminogruppe,
$R_5$ ein Wasserstoff- oder Chloratom,
$R_6$ eine Methylgruppe,
G eine n-Alkylengruppe mit 3 bis 5 Kohlenstoff-

OH
|
atomen oder eine $-CH-CO-$Gruppe oder auch, wenn B die $-CS-$Gruppe oder wenn

OH
|
A die $-CH-CO-$Gruppe darstellt, die $-CH_2CH_2-$Gruppe bedeuten, und deren Säureadditionssalze.

4. 1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propan
und dessen Säureadditionssalze.

5. 1-(1-Hydroxy-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propan
und dessen Säureadditionssalze.

6. 1-(7,8-Dimethoxy-1,3,4,5-tetrahydro-2H-3-benzazepin-2-on-3-yl)-3-[N-methyl-N-(3-(4-amino-3,5-dichlor-phenyl)-propyl)-amino]-propan
und dessen Säureadditionssalze.

7. Physiologisch verträgliche Säureadditionssalze der Verbindungen gemäss den Ansprüchen 1 bis 6.

8. Arzneimittel, enthaltend eine Verbindung gemäss den Ansprüchen 1 bis 6, oder deren physiologisch verträgliches Säureadditionssalz gemäss Anspruch 7 neben einem oder mehreren inerten Trägerstoffen und/oder Verdünnungsmitteln.

9. Verwendung einer Verbindung gemäss den Ansprüchen 1 bis 6 oder deren physiologisch verträgliches Säureadditionssalz gemäss Anspruch 7 zur Herstellung eines Arzneimittels, welches zur Behandlung von Sinustachykardien und zur Prophylaxe bzw. Therapie ischämischer Herzerkrankungen geeignet ist.

10. Verfahren zur Herstellung der Verbindungen gemäss den Ansprüchen 1 bis 7, dadurch gekennzeichnet, dass

a) eine Verbindung der allgemeinen Formel II

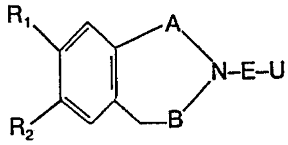

(II)

mit einer Verbindung der allgemeinen Formel III

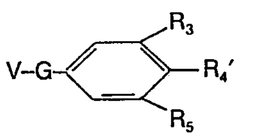

(III)

in denen

$R_1$, $R_2$, $R_3$, $R_5$, A, B, E und G wie in den Ansprüchen 1 bis 6 definiert sind und

$R_4'$ eine durch einen Schutzrest geschützte Amino- oder Methylaminogruppe darstellt oder die für $R_4$ in den Ansprüchen 1 bis 6 erwähnten Bedeutungen besitzt,

einer der Reste U oder V die $R_6$-NH-Gruppe darstellt, wobei $R_6$ wie in den Ansprüchen 1 bis 6 definiert ist, und

der andere der Reste U oder V eine nukleophile Austrittsgruppe wie ein Halogenatom oder eine Sulfonyloxygruppe darstellt, umgesetzt und gegebenenfalls anschliessend ein verwendeter Schutzrest abgespalten wird oder

b) zur Herstellung einer Verbindung der allgemeinen Formel I, in der A eine $-CH_2-CH_2-$, $-CH{=}CH-$, -COCO- oder $-N{=}CH$-Gruppe und B eine $-CH_2-$ oder -CO-Gruppe darstellen, eine Verbindung der allgemeinen Formel IV

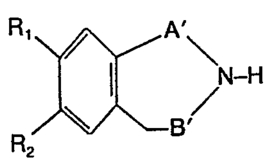

(IV)

in der

$R_1$ und $R_2$ wie in den Ansprüchen 1 bis 6 definiert sind,

A' eine $-CH_2-CH_2-$, $-CH{=}CH-$, -COCO- oder $-N{=}CH$-Gruppe und

B' eine $-CH_2-$ oder -CO-Gruppe bedeuten, mit einer Verbindung der allgemeinen Formel V

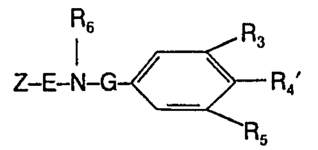

(V)

in der

$R_3$, $R_5$, $R_6$, E und G wie in den Ansprüchen 1 bis 6 definiert sind,

$R_4'$ eine durch eine Schutzgruppe geschützte Amino- oder Methylaminogruppe darstellt oder die für $R_4$ in den Ansprüchen 1 bis 6 erwähnten Bedeutungen besitzt und

Z eine nukleophile Austrittsgruppe wie ein Halogenatom oder eine Sulfonyloxygruppe bedeutet, umgesetzt und gegebenenfalls anschliessend ein verwendeter Schutzrest abgespalten wird oder

c) zur Herstellung einer Verbindung der allgemeinen Formel I, in der A keine $-\overset{NH_2}{\overset{|}{C}}H-CO$-Gruppe darstellt, eine Verbindung der allgemeinen Formel VI

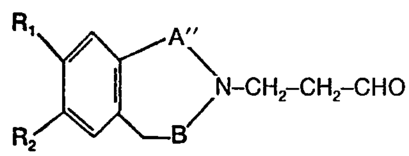

(VI)

in der

B, $R_1$ und $R_2$ wie in den Ansprüchen 1 bis 6 definiert sind, und

A'' eine $-CH_2-CH_2-$, $-CH{=}CH-$, $-N{=}CH-$, $-\overset{OH}{\overset{|}{C}}H-CO-$ oder $-\overset{NOH}{\overset{\|}{C}}-CO$-Gruppe darstellt, mit einem Amin der allgemeinen Formel VII

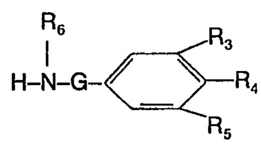

(VII)

in der

G und $R_3$ bis $R_6$ wie in den Ansprüchen 1 bis 6 definiert sind, in Gegenwart eines Reduktionsmittels umgesetzt wird oder

d) zur Herstellung einer Verbindung der allgemeinen Formel I, in der A keine $-\overset{NH_2}{\overset{|}{C}}H-CO$-Gruppe darstellt, eine Verbindung der allgemeinen Formel VIII

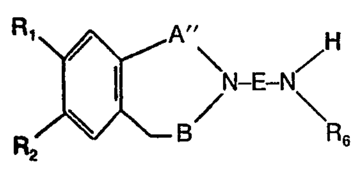

(VIII)

in der

B, E, $R_1$, $R_2$ und $R_6$ wie in den Ansprüchen 1 bis 6 definiert sind und

A″ eine $-CH_2-CH_2-$, $-CH=CH-$, $-N=CH-$,

OH NOH
| ‖
–CH–CO- oder –C–CO-Gruppe darstellt, mit einer Verbindung der allgemeinen Formel IX

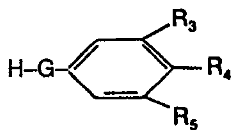
(IX)

in der

$R_3$ bis $R_5$ und G wie in den Ansprüchen 1 bis 6 definiert sind, wobei jedoch im Rest G die endständige $-CH_2$-Gruppe durch eine Carbonylgruppe ersetzt ist, in Gegenwart eines Reduktionsmittels umgesetzt wird oder

e) zur Herstellung einer Verbindung der allgemeinen Formel I, in der A die $-CH_2-CH_2$-Gruppe und B die Methylen- oder Carbonylgruppe darstellen, eine Verbindung der allgemeinen Formel X

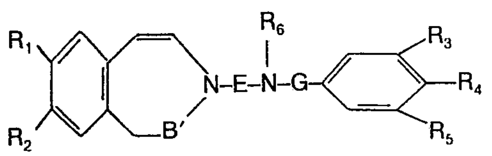
(X)

in der

$R_1$ bis $R_6$, E und G wie in den Ansprüchen 1 bis 6 definiert sind und

B′ die Methylen- oder Carbonylgruppe darstellt, hydriert wird oder

f) zur Herstellung einer Verbindung der allgemeinen Formel I, in der B die –CS-Gruppe darstellt, eine Verbindung der allgemeinen Formel XI

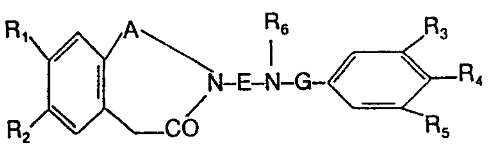
(XI)

in der

$R_1$ bis $R_6$, A, E und G wie in den Ansprüchen 1 bis 6 definiert sind, mit einem schwefeleinführenden Mittel umgesetzt wird oder

g) zur Herstellung einer Verbindung der allgemeinen Formel I, in der A eine

OH
|
–CH–CO-Gruppe darstellt, eine Verbindung der allgemeinen Formel XII

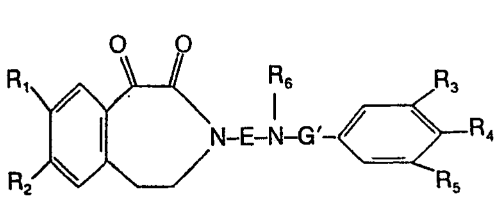
(XII)

in der

$R_1$ bis $R_6$ und E wie in den Ansprüchen 1 bis 6 definiert sind und

G′ eine n-Alkylengruppe mit 2 bis 5 Kohlenstoffatomen, in der eine Methylengruppe durch eine Carbonylgruppe ersetzt sein kann, oder eine Methylen-n-hydroxyalkylengruppe mit 1 bis 3 Kohlenstoffatomen im Alkylenteil, wobei die Methylengruppe mit dem Stickstoffatom verknüpft ist, darstellt, reduziert wird oder

h) zur Herstellung einer Verbindung der allgemeinen Formel I, in der A eine

NH₂
|
–CH–CO- oder

NOH
‖
–C–CO-Gruppe darstellt, eine Verbindung der allgemeinen Formel XIII

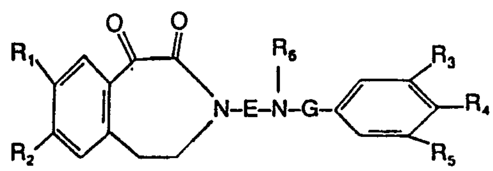
(XIII)

in der

$R_1$ bis $R_6$, E und G wie in den Ansprüchen 1 bis 6 definiert sind, mit einer Verbindung der allgemeinen Formel XIV

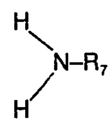
(XIV)

in der

$R_7$ ein Wasserstoffatom oder die Hydroxygruppe darstellt, umgesetzt und gegebenenfalls eine so erhaltene Verbindung anschliessend reduziert wird oder

i) zur Herstellung einer Verbindung der allgemeinen Formel I, in der A die $-N=CH$-Gruppe und $R_6$ eine Methylgruppe darstellen, eine Verbindung der allgemeinen Formel XV

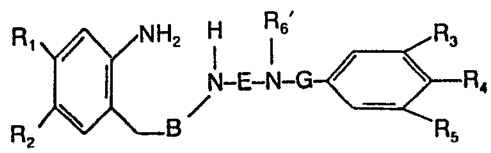
(XV)

in der

$R_1$ bis $R_5$, B, E und G wie in den Ansprüchen 1 bis 6 definiert sind und

$R_6'$ eine Methylgruppe darstellt, wobei gegebenenfalls vorhandene $NH_2$- oder –NH-Gruppen geschützt sein können, mit einem Orthoameisensäureester umgesetzt und anschliessend ein gegebenenfalls verwendeter Schutzrest abgespalten wird oder

k) zur Herstellung einer Verbindung der allgemeinen Formel I, in der A keine –COCO-Gruppe und G eine Methylen-n-hydroxy-alkylengruppe darstellt, eine Verbindung der allgemeinen Formel XVI

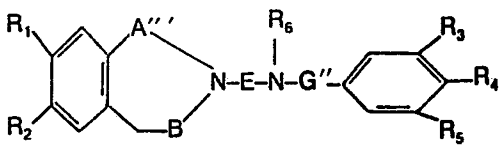

(XVI)

in der

$R_1$ bis $R_6$, B und E wie in den Ansprüchen 1 bis 6 definiert sind,

A‴ mit Ausnahme der –COCO-Gruppe die für A in den Ansprüchen 1 bis 6 erwähnten Bedeutungen besitzt und

G″ eine Methylen-n-alkylengruppe darstellt, wobei der Alkylenteil 1 bis 3 Kohlenstoffatome enthält und eine Methylengruppe des Alkylenteils durch eine Carbonylgruppe ersetzt ist, reduziert wird oder

l) zur Herstellung einer Verbindung der allgemeinen Formel I, in der $R_3$ eine Nitrogruppe und $R_4$ eine Aminogruppe darstellen, eine Verbindung der allgemeinen Formel XVII

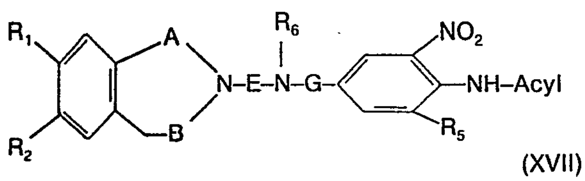

(XVII)

in der

$R_1$, $R_2$, $R_5$, $R_6$, A, B, E und G wie in den Ansprüchen 1 bis 6 definiert sind und

Acyl einen Acylrest darstellt, hydrolysiert wird oder

m) zur Herstellung einer Verbindung der allgemeinen Formel I, in der A eine $-CH_2-CH_2-$, $-CH=CH-$, $-N=CH-$ oder –COCO-Gruppe und einer der Reste $R_3$ oder $R_4$ eine Methoxy-, Methylamino- oder Dimethylaminogruppe oder $R_6$ eine Methylgruppe darstellen, eine Verbindung der allgemeinen Formel XVIII

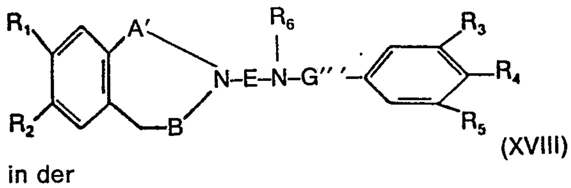

(XVIII)

in der

$R_1$ bis $R_6$, B und E wie in den Ansprüchen 1 bis 6 definiert sind, wobei jedoch mindestens einer der Reste $R_1$ bis $R_4$ eine Hydroxygruppe oder $R_4$ eine Amino- oder Methylaminogruppe oder $R_6$ ein Wasserstoffatom darstellen muss,

A′ eine $-CH_2-CH_2-$, $-CH=CH-$, $-N=CH-$ oder –COCO-Gruppe und

G‴ eine n-Alkylengruppe mit 2 bis 5 Kohlenstoffatomen darstellt, mit einer Verbindung der allgemeinen Formel XIX

$$CH_3-X \quad (XIX)$$

in der

X eine nukleophile Austrittsgruppe wie ein Halogenatom oder eine Sulfonyloxygruppe oder, falls mindestens einer der Reste $R_1$ bis $R_4$ eine Hydroxygruppe darstellt, X zusammen mit einem Wasserstoffatom des $CH_3$-Restes eine Diazogruppe oder auch, falls $R_6$ ein Wasserstoffatom oder $R_4$ eine Amino- oder Methylaminogruppe darstellen, ein Sauerstoffatom bedeuten, umgesetzt wird oder

n) zur Herstellung einer Verbindung der allgemeinen Formel I, in der A die –COCO-Gruppe und G eine n-Alkylengruppe mit 2 bis 5 Kohlenstoffatomen darstellen, eine Verbindung der allgemeinen Formel XX

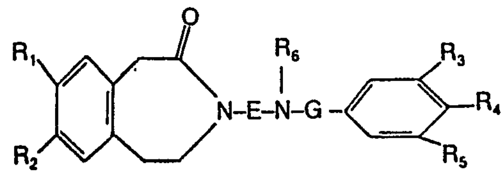

(XX)

in der

$R_1$ bis $R_6$, E und G wie in den Ansprüchen 1 bis 6 definiert sind, oxidiert wird oder

o) zur Herstellung einer Verbindung der allgemeinen Formel I, in der A die $-CH_2-CH_2-$Gruppe, B eine CO-Gruppe und G eine n-Alkylengruppe mit 2 bis 5 Kohlenstoffatomen darstellen, ein Benzazepin der allgemeinen Formel XXI

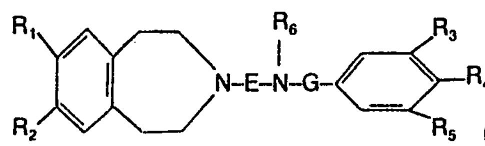

(XXI)

in der

$R_1$ bis $R_6$, E und G wie in den Ansprüchen 1 bis 6 definiert sind, mit Ruthenium-tetroxid oxidiert wird oder

p) zur Herstellung einer Verbindung der allgemeinen Formel I, in der A die $-CH_2-CH_2-$Gruppe und B die CO-Gruppe darstellen, eine Verbindung der allgemeinen Formel XXII

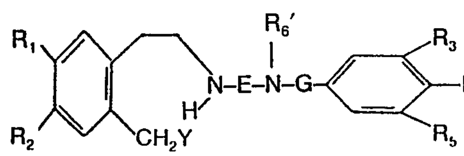

(XXII)

in der

$R_1$ bis $R_5$, G und E wie in den Ansprüchen 1 bis 6 definiert sind,

$R_6'$ eine Methylgruppe und

Y eine für den Ringschluss geeignete Gruppe darstellt, cyclisiert wird und

gewünschtenfalls anschliessend eine so erhaltene Verbindung der allgemeinen Formel I in ihr Säureadditionssalz, insbesondere in ihr physiologisch verträgliches Säureadditionssalz übergeführt wird.

**Claims**

1. Benzazepine derivatives of general formula I,

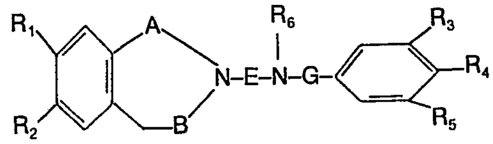

(I)

wherein

A represents a $-CH_2-CH_2-$ or $-CH=CH$ group and

B represents a -CS- group, or, if

G represents a n-alkylene group with 3 to 5 carbon atoms or a methylene-n-hydroxyalkylene group with 1 to 3 carbon atoms in the alkylene moiety, the methylene group being attached to the nitrogen atom, B may also represent a -CO group, or A represents a

$$\underset{x}{-N=CH-},\ \underset{x}{-\overset{OH}{\overset{|}{C}}H-CO-},\ \underset{x}{-\overset{N-OH}{\overset{\|}{C}}-CO-}\ \text{or}\ \underset{x}{-\overset{NH_2}{\overset{|}{C}}H-CO-}$$

group or else the -COCO-group, if

G represents an n-alkylene group with 3 to 5 carbon atoms or a methylene-n-hydroxyalkylene group with 1 to 3 carbon atoms in the alkylene moiety, the methylene group being attached to the nitrogen atom and

B represents a $-CH_2$-group, whilst the carbon atom marked x is linked to the phenyl nucleus,

E represents a n-propylen group,

G represents an n-alkylene group with 2 to 5 carbon atoms or a methylene-n-hydroxyalkylene group with 1 to 3 carbon atoms in the alkylene moiety, the methylene group being attached to the nitrogen atom,

$R_1$ and $R_2$ each represents a methoxy group,

$R_3$ represents a hydrogen, fluorine, chlorine or bromine atom, or a methoxy or nitro group,

$R_4$ represents a hydrogen atom, a methoxy, amino, methylamino or dimethylamino group,

$R_5$ represents a hydrogen, chlorine or bromine atom and

$R_6$ represents a hydrogen atom or a methyl group, and

1-(7,8-dimethoxy-1,3,4,5-tetrahydro-2H-3-benzazepin-2-on-3-yl)-3-[N-methyl-N-(2-phenylethyl)-amino]-propane and

1-(7,8-dimethoxy-1,3,4,5-tetrahydro-2H-3-benzazepin-2-on-3-yl)-3-[N-methyl-N-(2-(4-amino-3-nitro-phenyl)-ethyl)-amino]-propane

and the acid addition salts thereof.

2. Benzazepine derivatives of general formula I as claimed in claim 1, wherein

E is defined as in claim 1,

A represents a $-CH_2-CH_2$ group,

B represents a -CO- or -CS-group,

$R_1$ and $R_2$ each represents a methoxy group,

$R_3$ represents a hydrogen, fluorine, chlorine or bromine atom, or a methoxy group,

$R_4$ represents a methoxy, amino, methylamino or dimethylamino group,

$R_5$ represents a hydrogen, chlorine or bromine atom,

$R_6$ represents a methyl group, and

G represents an n-alkylene group with 3 to 5 carbon atoms or a $-CH_2-\overset{OH}{\overset{|}{C}}H$-group or, if B represents a -CS-group, G may also represent a $-CH_2-CH_2$-group, and the acid addition salts thereof.

3. Benzazepine derivatives of general formula I as claimed in claim 1, wherein

E is defined as in claim 1,

A represents a $-CH_2-CH_2$ group,

B represents a -CS-group or, if G represents an n-alkylene group with 3 to 5 carbon atoms, B may also represent a -CO-group or

A represents a $-\overset{OH}{\overset{|}{C}}H-CO$-group and

B represents a $-CH_2$-group,

$R_1$ and $R_2$ each represents a methoxy group,

$R_3$ represents a methoxy group, a hydrogen or chlorine atom,

$R_4$ represents a methoxy, amino, or dimethylamino group,

$R_5$ represents a hydrogen or chlorine atom,

$R_6$ represents a methyl group,

G represents an n-alkylene group with 3 to 5 carbon atoms or a $-CH_2-\overset{OH}{\overset{|}{C}}O$-group or, if B represents a -CS-group or if A represents a $-\overset{OH}{\overset{|}{C}}H-CO$-group, G may also represent a $-CH_2-CH_2$-group, and the acid addition salts thereof.

4. 1-(7,8-Dimethoxy-1,3,4,5-tetrahydro-2H-3-benzazepin-2-on-3-yl)-3-[N-methyl-N-(3-(3,4-dimethoxy-phenyl)-propyl)-amino]-propane

and the acid addition salts thereof.

5. 1-(1-Hydroxy-7,8-dimethoxy-1,3,4,5-tetrahydro-2H-3-benzazepin-2-on-3-yl)-3-[N-methyl-N-(2-(3,4-dimethoxy-phenyl)-ethyl)-amino]-propane

and the acid addition salts thereof.

6. 1-(7,8-Dimethoxy-1,3,4,5-tetrahydro-2H-3-benzazepin-2-on-3-yl)-3-[N-methyl-N-(3-(4-amino-3,5-dichloro-phenyl)-propyl)-amino]-propane

and the acid addition salts thereof.

7. Physiologically acceptable acid addition salts of the compounds as claimed in claims 1 to 6.

8. Pharmaceutical compositions containing a compound as claimed in claims 1 to 6 or a physiologically acceptable acid addition salt as claimed in claim 7 together with one or more inert carriers and/or diluents.

9. Use of a compound as claimed in claims 1 to 6 or a physiologically acceptable acid addition salt as claimed in claim 7 for the preparation of a pharmaceutical composition for the treatment of sinus tachycardia and for the prophylaxis or therapy of ischaemic disease.

10. Process for the preparing the compounds as claimed in claims 1 to 7 which comprises

a) reacting a compound of general formula II,

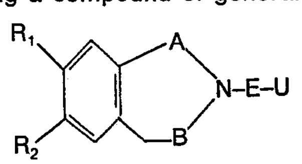

(II)

with a compound of general formula III,

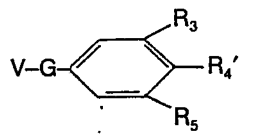

(III)

wherein

$R_1$, $R_2$, $R_3$, $R_5$, A, B, E and G are defined as in claims 1 to 6,

$R_4'$ represents an amino or methylamino group protected by a protecting group or has the meanings given for $R_4$ in the claims 1 to 6,

one of the groups U or V represents the $R_6$-NH group wherein $R_6$ is defined as in claims 1 to 6, and the

other group U or V represents a nucleophilically removable group such as a halogen atom or a sulphonyloxy group, and optionally subsequently splitting off any protecting group used or

b) in order to prepare a compound of general formula I, wherein A represents a $-CH_2-CH_2-$, $-CH=CH-$, $-COCO-$ or $-N=CH$ group and B represents a $-CH_2-$ or $-CO-$group, reacting a compound of general formula IV,

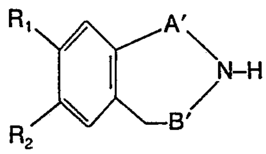

(IV)

wherein

$R_1$ and $R_2$ are defined as in claims 1 to 6,

A′ represents a $-CH_2-CH_2-$, $-CH=CH-$, $-COCO-$ or $-N=CH$ group and

B′ represents a $-CH_2-$ or $-CO-$ group, with a compound of the general formula V,

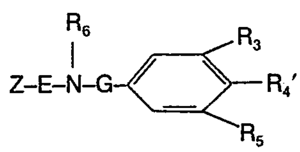

(V)

wherein

$R_3$, $R_5$, $R_6$, E and G are defined as in Claims 1 to 6,

$R_4'$ represents an amino or methylamino group protected by a protecting group or has the meanings given for $R_4$ in the claims 1 to 6 and

Z represents a nucleophilic leaving group such as a halogen atom or a sulphonyloxy group, and optionally subsequently splitting off any protecting group used or

c) in order to prepare compounds of general formula I, wherein

$NH_2$
|
A does not represent a $-CH-CO-$group, reacting a compound of general formula VI,

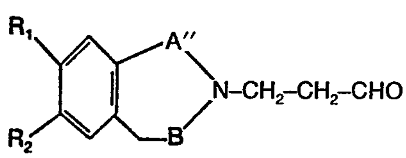

(VI)

wherein

B, $R_1$ and $R_2$ are defined as in claims 1 to 6 and A″ represents a $-CH_2-CH_2-$, $-CH=CH-$,

OH NOH
| ‖
$-N=CH-$, $-CH-CO-$ or $-C-CO-$group, with an amine of general formula VII,

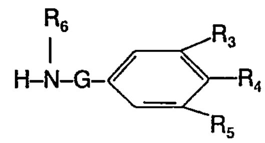

(VII)

wherein

G and $R_3$ to $R_6$ are defined as in claims 1 to 6, in the presence of a reducing agent or

d) in order to prepare compounds of general formula I, wherein A does not represent a

$NH_2$
|
$-CH-CO-$group, reacting a compound of general formula VIII,

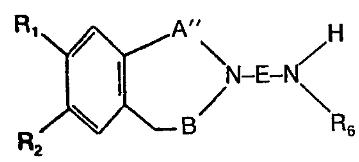

(VIII)

wherein

B, E, $R_1$, $R_2$ and $R_6$ are defined as in claims 1 to 6 and

A″ represents a $-CH_2-CH_2-$, $-CH=CH-$,

OH NOH
| ‖
$-N=CH-$, $-CH-CO-$ or $-C-CO-$group, with a compound of general formula IX,

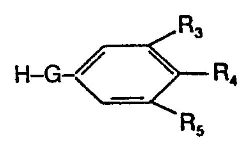

(IX)

wherein

$R_3$ to $R_5$ and G are defined as in claims 1 to 6 but in the group G a terminal $-CH_2-$group is replaced by a carbonyl group, in the presence of a reducing agent or

e) in order to prepare compounds of general formula I, wherein A represents a $-CH_2-CH_2-$group and B represents a methylene or carbonyl group, hydrogenating a compound of general formula X,

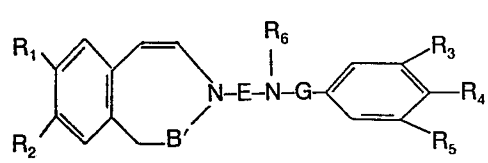

(X)

wherein

$R_1$ to $R_6$, E and G are defined as in claims 1 to 6 and

B′ represents a methylene or carbonyl group, or

f) in order to prepare compounds of general formula I, wherein B represents a –CS-group, reacting a compound of general formula XI,

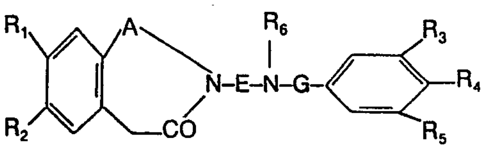
(XI)

wherein

$R_1$ to $R_6$, A, E and G are defined as in claims 1 to 6, with a sulphur-introducing agent or

g) in order to prepare compounds of general

OH
|
formula I, wherein A represents a –CH–CO-group, reducing a compound of general formula XII,

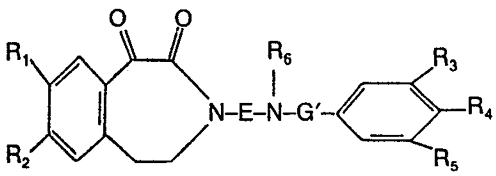
(XII)

wherein

$R_1$ to $R_6$ and E are defined as in claims 1 to 6 and

G′ represents an n-alkylene group with 2 to 5 carbon atoms, wherein a methylene group may be replaced by a carbonyl group, or a methylene-n-hydroxyalkylene group with 1 to 3 carbon atoms in the alkylene moiety, the methylene group being attached to the nitrogen atom, or

h) in order to prepare compounds of general formula I, wherein

NH2 NOH
| ‖
A represents a –CH–CO- or –C–CO-group, reacting a compound of general formula XIII,

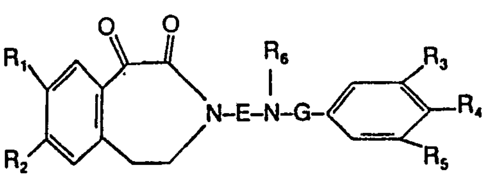
(XIII)

wherein

$R_1$ to $R_6$, E and G are defined as claims 1 to 6, with a compound of general formula XIV,

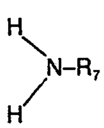
(XIV)

wherein

$R_7$ represents a hydrogen atom or a hydroxy group, and optionally a compound thus obtained is subsequently reduced, or

i) in order to prepare compounds of general formula I, wherein A represents an –N=CH-group and $R_6$ represents a methyl group, reacting a compound of general formula XV,

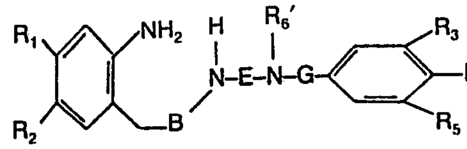
(XV)

wherein

$R_1$ to $R_5$, B, E and G are defined as in claims 1 to 6 and

$R_6'$ represents a methyl group, whereby optionally present $NH_2$- or NH-groups may be protected, with an orthformic acid ester and optionally subsequent splitting off of a protecting group used, or

k) in order to prepare compounds of general formula I, wherein A does not represent a –COCO-group and G represents a methylene-n-hydroxyalkylene group, reducing a compound of general formula XVI,

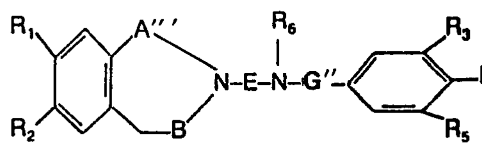
(XVI)

wherein

$R_1$ to $R_6$, B and E are defined as in claims 1 to 6,

A‴ has the meanings given for A in claims 1 to 6, with the exception of the –COCO-group, and

G″ represents a methylene-n-alkylene group, wherein the alkylene moiety contains 1 to 3 carbon atoms and a methylene group of the alkylene moiety is replaced by a carbonyl group, or

l) in order to prepare compounds of general formula I, wherein $R_3$ represents a nitro group and $R_4$ represents an amino group, hydrolysing a compound of general formula XVII,

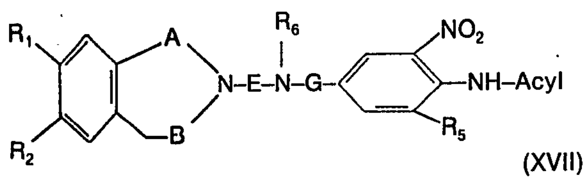
(XVII)

wherein

$R_1$, $R_2$, $R_5$, $R_6$, A, B, E and G are defined as in claims 1 to 6 and

Acyl represents an acyl group, or

m) in order to prepare compounds of general formula I, wherein A represents a $-CH_2-CH_2-$, –CH=CH-, –N=CH- or –COCO- group and one of the groups $R_3$ or $R_4$ represents a methoxy, methylamino or dimethylamino group or $R_6$ represents a methyl group, reacting a compound of general formula XVIII,

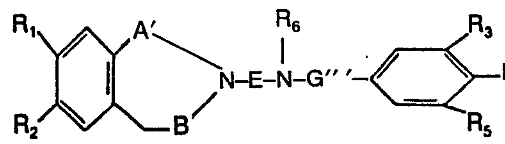
(XVIII)

wherein

$R_1$ to $R_6$, B and E are defined as in claims 1 to 6, but at least one of the groups $R_1$ to $R_4$ must represent a hydroxy group or $R_4$ must represent an

amino or methylamino group or $R_6$ must represent a hydrogen atom,

A′ represents a $-CH_2-CH_2-$, $-CH=CH-$, $-N=CH-$ or $-COCO-$group and

G‴ represents an n-alkylene group with 2 to 5 carbon atoms, with a compound of general formula XIX,

$$CH_3-X \quad (XIX)$$

wherein

X represents an nucleophilic leaving group such as a halogen atom or a sulphonyloxy group or, if at least one of the groups $R_1$ to $R_4$ represents a hydroxy group, X together with a hydrogen atom of the methyl group represents a diazo group or, if $R_6$ represents a hydrogen atom or $R_4$ represents an amino or methylamino group, X together with a hydrogen atom of the methyl group may also represent an oxygen atom, or

n) in order to prepare compounds of general formula I, wherein A represents a $-COCO-$group and G represents an n-alkylene group with 2 to 5 carbon atoms, oxidising a compound of general formula XX,

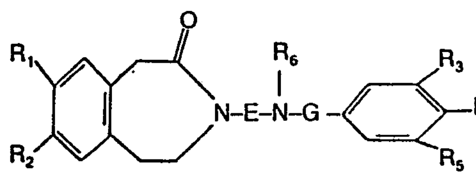

(XX)

wherein

$R_1$ to $R_6$, E and G are defined as in claims 1 to 6, or

o) in order to prepare compounds of general formula I, wherein A represents a $-CH_2CH_2-$group, B represents a $-CO-$group and G represents an n-alkylene group with 2 to 5 carbon atoms, oxidising a benzazepine of general formula XXI,

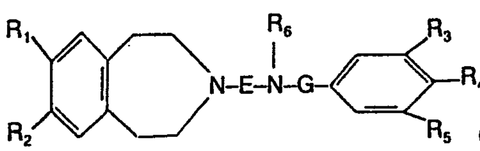

(XXI)

wherein

$R_1$ to $R_6$, E and G are defined as in claims 1 to 6, with ruthenium-tetroxide or

p) in order to prepare compounds of general formula I, wherein A represents a $-CH_2-CH_2-$group and B represents a $-CO-$group, oxidising a compound of general formula XXII,

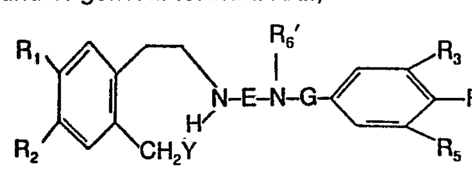

(XXII)

wherein

$R_1$ to $R_5$, G and E are defined as in claims 1 to 6,

$R_6'$ represents a methyl group and

Y represents a group suitable for cyclisation, and

subsequently, if desired, converting a compound of general formula I thus obtained into an acid addition salt thereof, more particularly a physiologically acceptable acid addition salt thereof.

## Revendications

1. Dérivés de benzazépine de formule générale I

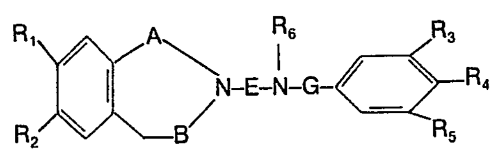

(I)

dans laquelle

A représente un groupe $-CH_2-CH_2-$ ou $-CH=CH-$ et

B le groupe $-CS-$ ou également, lorsque G représente un groupe n-alkylène avec 3 à 5 atomes de carbone ou un groupe méthylène-n-hydroxyalkylène avec 1 à 3 atomes de carbone dans la partie alkylène, le groupe méthylène étant lié à l'atome d'azote, le groupe $-CO-$ ou A représente un groupe

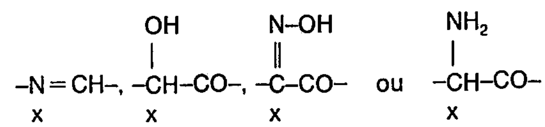

ou également le groupe $-COCO-$, lorsque

G représente un groupe n-alkylène avec 3 à 5 atomes de carbone ou un groupe méthylène-n-hydroxyalkylène avec 1 à 3 atomes de carbone dans la partie alkylène, le groupe méthylène étant lié à l'atome d'azote, et

B représente le groupe $-CH_2-$, l'atome de carbone matérialisé par x devant être chaque fois lié au noyau phényle,

E représente un groupe n-propylène,

G un groupe n-alkylène avec 2 à 5 atomes de carbone ou un groupe méthylène-n-hydroxyalkylène avec 1 à 3 atomes de carbone dans la partie alkylène, le groupe méthylène étant lié à l'atome d'azote,

$R_1$ et $R_2$ représentent chacun un groupe méthoxy,

$R_3$ représente un atome d'hydrogène, de fluor, de chlore ou de brome, un groupe méthoxy ou nitro,

$R_4$ un atome d'hydrogène, un groupe méthoxy, amino, méthylamino ou diméthylamino,

$R_5$ un atome d'hydrogène, de chlore ou de brome et

$R_6$ un atome d'hydrogène ou un groupe méthyle, ainsi que le

1-(7,8-diméthoxy-1,3,4,5-tétrahydro-2H-3-benzazépine-2-one-3-yl)-3-[N-méthyl-N-(2-phényléthyl)-amino]-propane et le

1-(7,8-diméthoxy-1,3,4,5-tétrahydro-2H-3-benzazépine-2-one-3-yl)-3-[N-méthyl-N-(2-(4-amino-3-nitrophényl)-amino]-propane

et leurs sels d'addition d'acides.

2. Dérivés de benzazépine de formule générale I selon la revendication 1, formule dans laquelle

E est défini comme dans la revendication 1,

A représente le groupe $-CH_2CH_2-$,

B le groupe -CO- ou -CS-,

$R_1$ et $R_2$ représetent chacun un groupe méthoxy,

$R_3$ représente un atome d'hydrogène, de fluor, de chlore ou de brome ou un groupe méthoxy,

$R_4$ un groupe méthoxy, amino, méthylamino ou diméthylamino,

$R_5$ un atome d'hydrogène, de chlore ou de brome,

$R_6$ le groupe méthyle et

G un groupe n-alkylène avec 3 à 5 atomes de carbone ou le groupe

OH
|
$-CH_2-CH-$

ou également, lorsque B représente le groupe -CS-, le groupe $CH_2-CH_2-$ et leurs sels d'addition d'acides.

3. Dérivés de benzazépine de formule générale I selon la revendication 1, formule dans laquelle

E est défini comme dans la revendication 1,

A représente le groupe $-CH_2-CH_2-$ et

B le groupe -CS- ou également, lorsque G représente un groupe n-alkylène avec 3 à 5 atomes de carbone, le groupe -CO- ou

A représente le groupe

OH
|
-CH-CO

et B le groupe $-CH_2-$,

$R_1$ et $R_2$ représentent chacun un groupe méthoxy,

$R_3$ représente un groupe méthoxy, un atome d'hydrogène ou de chlore,

$R_4$ un groupe méthoxy, amino ou diméthylamino,

$R_5$ un atome d'hydrogène ou de chlore,

$R_6$ un groupe méthyle,

G un groupe n-alkylène avec 3 à 5 atomes de carbone ou un groupe

OH
|
-CH-CO-

ou également, lorsque B représente le groupe -CS- ou que A représente le groupe

OH
|
-CH-CO-,

le groupe $-CH_2CH_2-$, et leurs sels d'addition acides.

4. Le 1-(7,8-diméthoxy-1,3,4,5-tétrahydro-2H-3-benzazépine-2-one-3-yl)-3-[N-méthyl-N-(3-(3,4-diméthoxy-phényl)-propyl)-amino]-propane

et ses sels d'addition d'acides.

5. Le 1-(1-hydroxy-7,8-diméthoxy-1,3,4,5-tétrahydro-2H-3-benzazépine-2-one-3-yl)-3-[N-méthyl-N-(2-(3,4-diméthoxyphényl)-éthyl)-amino]-propane

et ses sels d'addition d'acides.

6. Le 1-(7,8-diméthoxy-1,3,4,5-tétrahydro-2H-3-benzazépine-2-one-3-yl)-3-[N-méthyl-N-(3-(4-amino-3,5-dichlorophényle)-propyl)-amino]-propane

et ses sels d'addition d'acides.

7. Des sels d'addition d'acides des composés selon les revendications 1 à 6 physiologiquement compatibles.

8. Médicament contenant, outre un ou plusieurs excipients inertes et/ou diluants, un composé selon les revendications 1 à 6 ou son sel d'addition d'acide physiologiquement compatible selon la revendication 7.

9. Utilisation d'un composé selon les revendications 1 à 6 ou de son sel d'addition d'acide physiologiquement compatible selon la revendication 7 pour la préparation d'un médicament qui est adapté au traitement des tachycardies sinusales et à la prévention, respectivement à la thérapie de cardiopathies ischémiques.

10. Procédé de préparation de composés selon les revendications 1 à 7, caractérisé en ce que

a) on fait réagir un composé de formule générale II

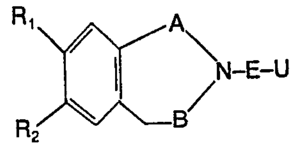

(II)

avec un composé de formule générale III

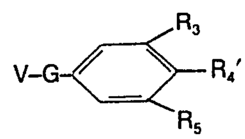

(III)

formules dans lesquelles

$R_1$, $R_2$, $R_3$, $R_5$, A, B, E et G sont définis comme dans les revendications 1 à 6 et

$R_4'$ représente un groupe amino ou méthylamino protégé par un reste protecteur ou a les significations précisées à propos de $R_4$ dans les revendications 1 à 6,

un des restes U ou V représente le groupe $R_6$-NH-, $R_6$ étant défini comme dans les revendications 1 à 6, et

l'autre des restes U ou V représente un groupe d'élimination nucléophile tel qu'un atome d'halogène ou un groupe sulfonyloxy et que, le cas échéant, le reste protecteur employé est ensuite éliminé ou

b) que, pour la préparation d'un composé de formule générale I dans laquelle A représente un groupe $-CH_2-CH_2-$, $-CH=CH-$, -COCO- ou $-N=CH-$ et B représente un groupe $-CH_2-$ ou -CO-, on fait réagir un composé de formule générale IV

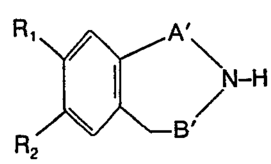

(IV)

dans laquelle

$R_1$ et $R_2$ sont définis comme dans les revendications 1 à 6,

A' représente un groupe $-CH_2CH_2$, $-CH=CH-$, -COCO- ou $-N=CH-$ et

B' un groupe $-CH_2-$ ou -CO-, avec un composé de formule générale V

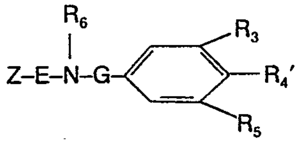 (V)

dans laquelle
$R_3$, $R_5$, $R_6$, E et G sont définis comme dans les revendications 1 à 6,
$R_4'$ représente un groupe amino ou méthylamino protégé par un groupe protecteur ou a les significations précisées à propos de $R_4$ dans les revendications 1 à 6 et Z représente un groupe d'élimination nucléophile tel qu'un atome d'halogène ou un groupe sulfonyloxy et que, le cas échéant, le reste protecteur utilisé est ensuite éliminé ou

c) que, pour la préparation d'un composé de formule générale I dans laquelle A ne représente

NH$_2$
|
pas de groupe –CH–CO–, on fait réagir en présence d'un agent de réduction un composé de formule générale VI

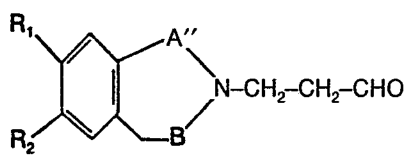 (VI)

dans laquelle
B, $R_1$ et $R_2$ sont définis comme dans les revendications 1 à 6 et
A″ représente un groupe $-CH_2-CH_2-$, –CH = CH–,

OH NOH
| ||
–N = CH–, –CH–CO– ou –C–CO–, avec une amine de formule générale VII

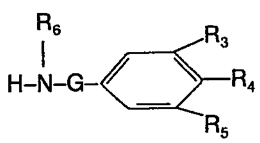 (VII)

dans laquelle
G et $R_3$ à $R_6$ sont définis comme dans les revendications 1 à 6 ou

d) que, pour la préparation d'un composé de formule générale I dans laquelle A ne représente

NH$_2$
|
pas de groupe –CH–CO–, on fait réagir en présence d'un agent de réduction un composé de formule générale VIII

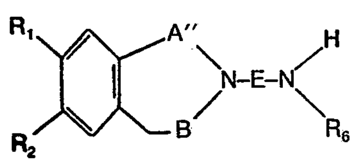 (VIII)

dans laquelle
B, E, $R_1$, $R_2$ et $R_6$ sont définis comme dans les revendications 1 à 6 et
A″ représente un groupe $-CH_2-CH_2-$, –CH = CH–,

OH NOH
| ||
–N = CH–, –CH–CO– ou –C–CO–, avec un composé de formule générale IX

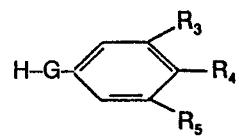 (IX)

dans laquelle
$R_3$ à $R_5$ et G sont définis comme dans les revendications 1 à 6, le groupe $-CH_2-$ terminal dans le reste G étant toutefois remplacé par un groupe carbonyle ou

e) que, pour la préparation d'un composé de formule générale I dans laquelle A représente le groupe $-CH_2-CH_2-$ et B le groupe méthylène ou carbonyle, un composé de formule générale X

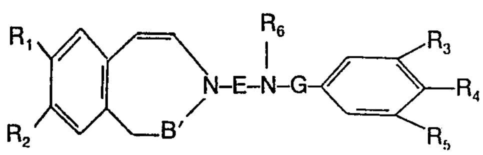 (X)

dans laquelle
$R_1$ à $R_6$, E et G sont définis comme dans les revendications 1 à 6 et
B′ représente le groupe méthylène ou carbonyle, est hydrogène ou

f) que, pour la préparation d'un composé de formule générale I dans laquelle B représente le groupe –CS–, un composé de formule générale XI

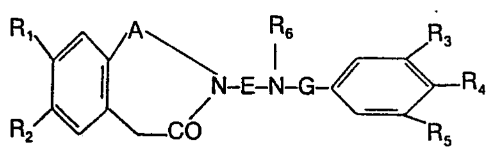 (XI)

dans laquelle
$R_1$ à $R_6$, A, E et G sont définis comme dans les revendications 1 à 6, est mis à réagir avec un agent introduisant du souffre ou

g) que, pour la préparation d'un composé de formule générale I, dans laquelle A représente un

OH
|
groupe –CH–CO–, un composé de formule générale XII

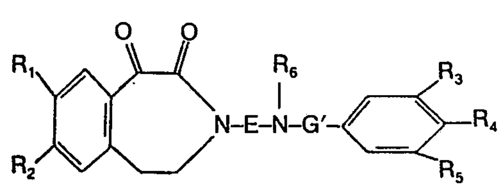 (XII)

dans laquelle

$R_1$ à $R_6$ et E sont définis comme dans les revendications 1 à 6 et

G′ représente un groupe n-alkylène avec 2 à 5 atomes de carbone dans lequel un groupe méthylène peut être remplacé par un groupe carbonyle ou un groupe méthylène-n-hydroxyalkylène avec 1 à 3 atomes de carbone dans la partie alkylène, le groupe méthylène étant lié à l'atome d'azote, est réduit ou

h) que, pour la préparation d'un composé de formule générale I, dans laquelle A représente un

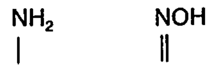

groupe –CH–CO- ou –C–CO-, un composé de formule générale XIII

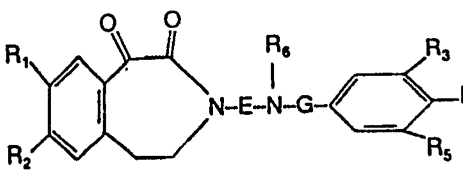

(XIII)

dans laquelle

$R_1$ à $R_6$, E et G sont définis comme dans les revendications 1 à 6, est mis à réagir avec un composé de formule générale XIV

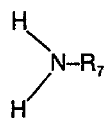

(XIV)

dans laquelle

$R_7$ représente un atome d'hydrogène ou le groupoe hydroxy et qu'éventuellement un composé obtenu de cette façon est consécutivement réduit ou

i) que, pour la préparation d'un composé de formule I dans laquelle A représente le groupe $-N=CH-$ et $R_6$ un groupe méthyle, un composé de formule générale XV

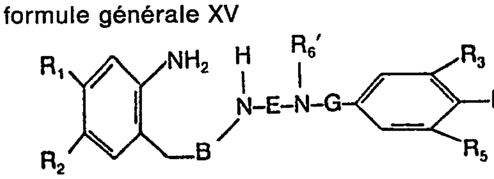

(XV)

dans laquelle

$R_1$ à $R_5$, B, E et G sont définis comme dans les revendications 1 à 6 et

$R_6'$ représente un groupe méthyle, les groupes $NH_2$- ou –NH- éventuellement présents pouvant être protégés, est mis à réagir avec un orthoformiate et qu'ensuite un reste protecteur éventuellement utilisé est éliminé ou

k) que, pour la préparation d'un composé de formule générale I dans laquelle A ne représente pas de groupe -COCO- et G représente un groupe méthylène-n-hydroxyalkylène, un composé de formule générale XVI

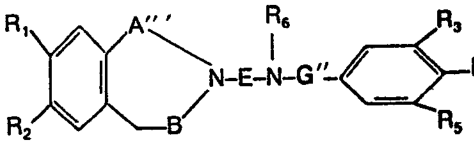

(XVI)

dans laquelle

$R_1$ à $R_6$, B et E sont définis comme dans les revendications 1 à 6,

A‴ possède, exception faite du groupe –COCO–, les significations précisées à propos de A dans les revendications 1 à 6 et

G″ représente un groupe méthylène-n-alkylène, la partie alkylène contenant 1 à 3 atomes de carbone et un groupe méthylène de la partie alkylène étant remplacé par un groupe carbonyle, est réduit ou

l) que, pour la préparation d'un composé de formule générale I, dans laquelle $R_3$ représente un groupe nitro et $R_4$ un groupe amino, un composé de formule générale XVII

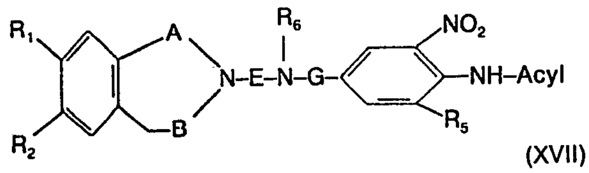

(XVII)

dans laquelle

$R_1$, $R_2$, $R_5$, $R_6$, A, B, E et G sont définis comme dans les revendications 1 à 6 et

acyle représente un reste acyle, est hydrolysé ou

m) que, pour la préparation d'un composé de formule générale I dans laquelle A représente un groupe $-CH_2-CH_2-$, $-CH=CH-$, $-N=CH-$ ou –COCO-, un des restes $R_3$ ou $R_4$ représente un groupe méthoxy, méthyle, amino ou diméthylamino ou $R_6$ représente un groupe méthyle, on fait réagir un composé de formule générale XVIII

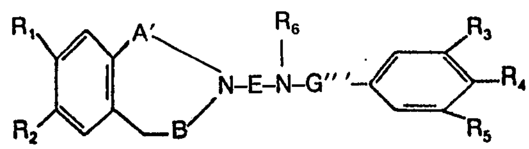

(XVIII)

dans laquelle

$R_1$ à $R_6$, B et E sont définis comme dans les revendications 1 à 6, au moins un des restes $R_1$ à $R_4$ devant toutefois représenter un groupe hydroxy ou $R_4$ un groupe amino ou méthylamino ou $R_6$ un atome d'hydrogène, A′ représente un groupe $-CH_2-CH_2-$, $-CH=CH-$, $-N=CH-$ ou –COCO- et

G‴ représente un groupe n-alkylène avec 2 à 5 atomes de carbone, avec un composé de formule générale XIX

$$CH_3-X \qquad (XIX)$$

dans laquelle

X représente un groupe d'élimination nucléophile tel qu'un atome d'halogène ou un groupe sulfonyloxy ou, dans le cas où au moins un des restes $R_1$ à $R_4$ représente un groupe hydroxy, X représente avec un atome d'hydrogène du reste $CH_3$- un groupe diazo ou également, au cas où $R_6$ représente un atome d'hydrogène ou $R_4$ représente un groupe amino ou méthylamino, X représente un atome d'oxygène ou

n) que, pour la préparation d'un composé de formule générale I dans laquelle A représente le groupe –COCO- et G un groupe n-alkylène avec 2 à 5 atomes de carbone, un composé de formule générale XX

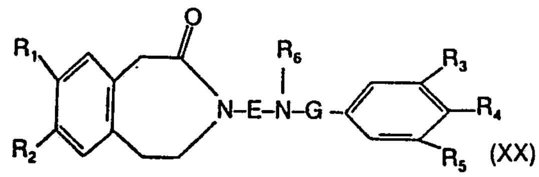

(XX)

dans laquelle

$R_1$ à $R_6$, E et G sont définis comme dans les revendications 1 à 6, est oxydé ou

o) que, pour la préparation d'un composé de formule générale I dans laquelle A représente le groupe $-CH_2-CH_2-$, B un groupe CO- et G un groupe n-alkylène avec 2 à 5 atomes de carbone, une benzazépine de formule générale XXI

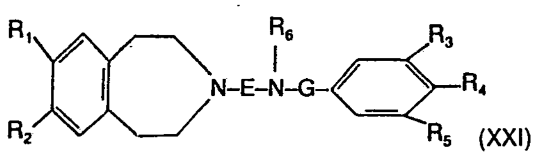

(XXI)

dans laquelle

$R_1$ à $R_6$, E et G sont définis comme dans les revendications 1 à 6, est oxydé avec un tétroxyde de ruthénium ou

p) que, pour la préparation d'un composé de formule générale I dans laquelle A représente le groupe $-CH_2-CH_2-$ et B le groupe CO-, un composé de formule générale XXII

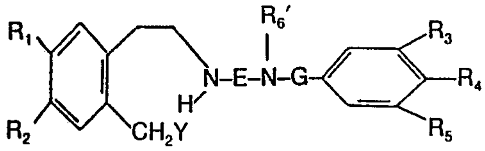

(XXII)

dans laquelle

$R_1$ à $R_6$, G et E sont définis comme dans les revendications 1 à 6,

$R_6'$ représente un groupe méthyle et

Y un groupe se prêtant à la cyclisation, est cyclisé et que, si on le désire, un composé de formule générale I ainsi obtenu est ensuite transformé en son sel d'addition d'acide, notamment en son sel d'addition d'acide physiologiquement compatible.